# EUROPEAN PATENT SPECIFICATION

(11) **EP 0 706 792 B1**
(45) Date of publication and mention of the grant of the patent: **12.11.2003**
(21) Application number: 95112851.1
(22) Date of filing: 20.03.1989
(51) Int. Cl.: A61K 9/50, A61K 39/00

(54) **Method of potentiating an immune response and compositions therefor**
Verfahren zur Potenzierung einer Immunantwort sowie die dazugehörigen Mittel
Procédé de potentialisation d'une réponse immunitaire et les compositions correspondantes

(30) Priority: 18.03.1988 US 169973
(43) Date of publication of application: 17.04.1996
(62) Divisional of application: 89302746.6
(73) Proprietor: THE UAB RESEARCH FOUNDATION, Birmingham Alabama 35294 (US); SOUTHERN RESEARCH INSTITUTE, Birmingham Alabama 35255-5305 (US)
(72) Inventor: Tice, Thomas T., Birmingham, Alabama 35244 (US); Eldridge, John H., Birmingham, Alabama 35120 (US); Gilley, Richard M., Birmingham, Alabama 35243 (US); Stass, Jay K., Birmingham, Alabama 35120 (US)
(74) Representative: Leissler-Gerstl, Gabriele

(56) References cited:
- EP-A- 0 257 915
- EP-A- 0 266 119
- WO-A-84/00294
- WO-A-87/03197
- WO-A-88/01213
- FR-A- 2 304 326
- GB-A- 2 160 312

## Description

This invention relates to a formulation for administering an immunogenic agent encapsulated in one or more biocompatible polymer or copolymer excipients, preferably a biodegradable polymer or copolymer. The immunogenic agent is encapsulated as microcapsules, which due to their proper size and physicalchemical properties, reach and are effectively taken up by the folliculli lymphatic aggregati, otherwise known as the "Peyer's patches", of the gastrointestinal tract in an animal without loss of effectiveness due to the agent having passed through the gastrointestinal tract. Similar folliculli lymphatic aggregati can be found in the respiratory tract, genitourinary tract, large intestine and other mucosal tissues of the body. Hereafter, the above-described tissues are referred to in general as mucosally-associated lymphoid tissues.

The use of microencapsulation to protect sensitive bioactive agents from degradation has become well-known. Typically, a bioactive agent is encapsulated within any of a number of protective wall materials, usually polymeric in nature. The agent to be encapsulated can be coated with a single wall of polymeric material (microcapsules), or can be homogeneously dispersed within a polymeric matrix (microspheres). (Hereafter, the term microcapsules refers to both microcapsules and microspheres and the terms "encapsulation" and "microencapsulation" should be construed accordingly). The amount of agent inside the microcapsule can be varied as desired, ranging from either a small amount to as high as 95% or more of the microcapsule composition. The diameter of the microcapsule can also be varied as desired, ranging from less than one micrometer to as large as three millimeters or more.

Peyer's patches are aggregates of lymphoid nodules located in the wall of the small intestine, large intestine and appendix and are an important part of body's defense against the adherence and penetration of infectious agents and other substances foreign to the body. Antigens are substances that induce the antibody-producing and/or cell-mediated immune systems of the body, and include such things as foreign protein or tissue. The immunologic response induced by the interaction of an antigen with the immune system nay be either positive or negative with respect to the body's ability to mount an antibody or cell-mediated immune response to a subsequent reexposure to the antigen. Cell-mediated immune responses include responses such as the killing of foreign cells or tissues, "cell-mediated cytoxicity", and delayed-type hypersensitivity reactions. Antibodies belong to a class of proteins called immunoglobulins (Ig), which are produced in response to an antigen, and which combine specifically with the antigen. When an antibody and antigen combine, they form a complex. This complex may aid in the clearance of the antigen from the body, facilitate the killing of living antigens such as infectious agents and foreign tissues or cancers, and neutralize the activity of toxins or enzymes. In the case of the mucosal surfaces of the body the major class of antibody present in the secretions which bathe these sites is secretory immunoglobulin A (sIgA). Secretory IgA antibodies prevent the adherence and penetration of infectious agents and other antigens to and through the mucosal tissues of the body.

While numerous antigens enter the body through the mucosal tissues, commonly employed immunization methods, such as intramuscular or subcutaneous injection of antigens or vaccines, rarely induce the appearance of sIgA antibodies in mucosal secretions. Secretory IgA antibodies are most effectively induced through direct immunization of the mucosally-associated lymphoid tissues, of which the Peyer's patches of the gastrointestinal tract represent the largest mass in the body.

Peyer's patches possess IgA precursor 8 cells which can populate the lamina propria regions of the gastrointestinal and upper respiratory tracts and differentiate into mature IgA synthesizing plasma cells. It is these plasma cells which actually secrete the antibody molecules. Studies by Heremans and Bazin measuring the development of IgA responses in mice orally immunized with antigen showed that a sequential appearance of antigen-specific IgA plasma cells occurred, first in mesenteric lymph nodes, later in the spleen, and finally in the lamina propria of the gastrointestinal tract (Bazin, H., Levi, G., and Doria, G. Predominant contribution of IgA antibody-forming cells to an immune response detected in extraintestinal lymphoid tissues of germ free mice exposed to antigen via the oral route. J. Immunol. 105:1049; 1970 and Crabbe, P.A., Nash, D.R., Bazin, It., Eyssen, H. and Heremans, J.F. Antibodies of the IgA type in intestinal plasma cells of germ-free nice after oral or parenteral immunization with ferritin. J. Exp. Med. 130:723; 1969). Subsequent studies have shown that oral administration of antigens leads to the production of sIgA antibodies in the gut and also in mucosal secretions distant to the gut, e.g., in bronchial washings, colostrum, milk, saliva and tears (Mestecky, J., McGhee, J.R., Arnold, R.R., Michalek, S.M., Prince, S.J. and Babb, J.L. Selective induction of an immune response in human external secretions by ingestion of bacterial antigen. J. Clin. Invest. 61:731; 1978, Montgomery, P.C., Rosner, B.R. and Cohen, J. The secretory antibody response. Anti-DNP antibodies induced by dinitrophenylated Type III pneumococcus. Immunol. Commun. 3:143; 1974, and Hanson, L.A., Ahistedt, S., Carlsson, B., Kaijser, B., Larsson, P., MattsbyBaltzer, A., Sohl Akerlund, A., Svanborg Eden, C. and Dvennerholm, A.M. Secretory IgA antibodies to enterobacterial virulence antigens: their induction and possible relevance, Adv. Exp. Med. Biol. 1007:165; 1978). It is apparent, therefore, that Peyer's patches are an enriched source of precursor IgA cells, which, subsequent to antigen sensitization, follow a circular migrational pathway and account for the expression of IgA at both the region of initial antigen exposure and at distant mucosal surfaces. This circular pattern provides a mucosal immune system by continually transporting sensitized B cells to mucosal sites for responses to gut-encountered environmental antigens and potential pathogens.

WO-A 87/03197 discloses a pharmaceutical composition comprising sodium cromoglycate for treating asthma hypersensitivity. Thus, this composition is directed to reducing an immune response in contrast to potentiating an immune response. There are other preparations known in the art which comprise a drug or medicament where an immune response is to be avoided rather than potentiated. This is disclosed inter alia in EP-A 0 257 915 describing a formulation for the treatment of asthma or in US-A 45 42 025 being concerned with a method for preparing a composition comprising microparticles which contain an anti inflammatory agent; where the microparticles preferably have a size of up to 200 µm. An article in "Biomedical Applications of Microencapsulation" Ed. F. Lim, chapter 3, on pages 53-72, is also concerned with drug loaded microspheres which can be administered parenterally. Another article by T.M.S. Chang discloses in Journal of Bioengineering, Vol. 1, pp. 25-32, 1976, to use semipermeable microcapsules for encapsulating biological active agents which are injected to release the agent in the blood stream. Artursson et al. in Journal of Pharmaceutical Sciences, Vol. 73, Nr. 11, pp 1507-1513 have studied the use of polyacryl starch microparticles as drug carrier systems which are used for injecting drugs. The composition disclosed in all these documents are not expected to induce an immune response which is the gist of the present invention.

Moreover, it was known in the art to prepare formulations to be administered via the oral route or by injection. For example EP-A 0 266 119 teaches about a method and a formulation for targeting a bioactive agent to the Peyer's patches in the gastro-intestinal tract; WO 88/01213 is concerned with delivery systems for pharmacological agents which are administered in proteinoid microspheres which deliver the agent either in the neutral blood stream or the gastro-intestinal tract. WO 84/00294 is directed to a crystallized carbohydrate matrix for the delayed release of a substance where a depot matrix is injected. These compositions do not adress the MALT.

Cox & Taubman in Int. Archs. Allergy appi. Immun. 75: 126-131 (1984) teach about the oral induction of a secretory antibody response by antigens and Cox & Muench in Int. Archs. Allergy appl. Immune. 74: 249-255 (1984) teach about the effect of introduction of an antigen by gastric intubation, i.e. via the oral route. Schröder & Stahl in Journal of Immunological Methods, 70 (1984) 127-132, disclose a method for preparing a matrix with entrapped antigen to be administered via an injection. EP-A 0 292 710 is a document being concerned with a process for preparing compositions comprising microcapsules which are produced by a special process where the microcapsules preferably have a size of 30 to 120 µm and are administered by injection. D.O'Hagan (1987) CRC Critical Reviews in Therapeutic Drug Carrier Systems, 4, 197-220 emphasizes on the oral administration and the intestinal absorption of particulate matter. An article of Kreuter & Liehl in Journal of Pharmaceutical Sciences, Vol 70, Nr. 4, pp 367-371 (1981) is concerned with the use of nanoparticles and nanocapsules to inject an antigen. However, all these formulations do not adress the MALT.

Furthermore, it was thought in the past that it is necessary to have an antigen on the surface to present it to the immune system to get an immune response. This was emphasized for example in FR-A 23 04 326, by Artursson et.al. in J. Pharm. Exp. Therap. 234, 255-260 (1985), by Cox & Taubman and Cox & Muench (cited above) and by Kreuter & Liehl. In contrast thereto, the present inventors found that an antigen can induce an immune response if it is encapsulated provided that the parameters of claim 1 are fulfilled.

Of particular importance to the present invention is the ability of oral immunization to induce protective antibodies. It is known that the ingestion of antigens by animals results in the appearance of antigen-specific sIgA antibodies in bronchial and nasal washings. For example, studies with human volunteers show that oral administration of influenza vaccine is effective at inducing secretory anti-influenza antibodies in nasal secretions.

There exists a need for a method to immunize through other mucosal tissues of the body than the gastrointestinal tract which overcomes the problems of degradation of the antigen and targets the delivery to the mucosally-associated lymphoid tissues. In addition, the need exists for the protection from degradation of mucosally applied immunogenic agents, improves and/or targets their entrance into the body through the mucosally-associated lymphoid tissues and releases the immunogenic agent once it has entered the body.

This invention is concerned with the use of biocompatible microcapsules comprising an immunogenic agent encapsulated in a biocompatible excipient and having a size of less than approximately 10 µm for preparing a nasal, rectal, ophthalmic, intratracheal or inhalation preparation for delivering the immunogenic agent to mucosally associated lymphoreticular tissue of a human or other animal. Preferred embodiments are outlined in claims 2 to 15.

Moreover, the present invention is concerned with a composition for stimulating the immune response of a human or other animal, comprising a mixture of a first amount of biocompatible microcapsules having a size of less than approximately 10 µm and containing an immunogenic agent encapsulated in a first biocompatible excipient and a second amount of biocompatible microcapsules having a size of greater than 10 µm and containing an immunogenic agent encapsulated in a second biocompatible excipient, the first microcapsules providing a primary immunological response and the second microcapsules releasing the agent contained in the second microcapsules in a pulsed manner to potentiate a subsequent immunological response.

Preferred embodiments of this composition are inter alia as outlined in claims 17 to 19.

Another object of the present invention is the use of a composition comprising a mixture of a first amount of biocompatible microcapsules having a size of less than 10 µm and containing an immunogenic agent encapsulated in a biocompatible excipient and a second amount of biocompatible microcapsules having a size of greater than 10 µm and containing an immunogenic agent encapsulated in a biocompatible excipient, the first microcapsules providing a primary immunological response and the second microcapsules releasing the agent contained in the second microcapsules in a pulsed manner, for preparing a preparation for inducing an immunological response in a human or other animal.

Another object of the present invention is the use for the preparation of a combined composition comprising first biocompatible microcapsules comprising an immunogenic agent encapsulated in a biocompatible excipient and having a size of between 1 µm and 10 µm for adminstration to a human or other animal by a first route and second biocomaptible microcapsules comprising the immunogenic agent encapsulated in a biocompatible excipient and having a size of between 1 µm and 10 µm for adminstration to a human or other animal by a second route different from the first route for potentiating the immune response of a human or other animal.

This invention relates inter alia to a formulation for targeting to and then releasing an immunogenic agent in the body of an animal by mucosal application, and in particular, intratracheal administration. The agent is microencapsulated in a biocompatible polymer or copolymer, preferably a biodegradable polymer or copolymer which is capable of existing on a mucosal surface without degradation or with minimal degradation so that the agent reaches and enters the mucosally-associated lymphoid tissues (MALT) unaltered and in effective amounts. The term biocompatible is defined as a polymeric material which is not toxic to the body, is not carcinogenic, and which should not induce inflammation in body tissues. It is preferred that the microcapsule polymeric excipient be biodegradable in the sense that it should degrade by bodily processes to products readily disposable by the body and should not accumulate in the body. The microcapsules are also of a size and physicalchemical composition capable of being effectively and selectively taken up by the MALT. Therefore, the problems of the agent reaching other mucosally-associated tissue and being taken up are solved.

It is a further object of this invention to provide a method of administering an antigen to an animal which results in the antigen reaching and being taken up by the mucosally-associated lymphoid tissues, and thereby stimulating the mucosal immune system, without losing its effectiveness as a result of degradation on the mucosal surface.

It is a still further object of this invention to provide a method of administering an antigen to an animal which results in the antigen being taken up by the mucosally-associated lymphoid tissues, and thereby stimulating the systemic immune system, without losing its effectiveness as a result of degradation on the mucosal surface.

It is a still further object of this invention to provide a method of administering an immunogenic agent to an animal which results in the agent reaching and being taken up by the mucosally-associated lymphoid tissues, and thereby resulting in an increased local or systemic concentration of the agent.

It is a still further object of this invention to provide a formulation consisting of a core immunogenic ingredient and an encapsulating polymer or copolymer excipient which is biocompatible and preferably biodegradable as well, which can be utilized in the mucosal-administration methods described above.

It is another object of this invention to provide an improved vaccine delivery system which obviates the need for immunopotentiators.

It is a still further object of this invention to provide an improved vaccine delivery system for the induction of immunity through the pulsatile release of antigen from a single administration of microencapsulated antigen.

It is a still further object of this invention to provide an improved vaccine delivery system which both obviates the need for immunopotentiators and affords induction of immunity through pulsatile releases of antigen all from a single administration of microcapsulated antigen.

It is a further object of this invention to provide a composition capable of achieving these above-referenced objects.

### BRIEF DESCRIPTION OF THE FIGURE

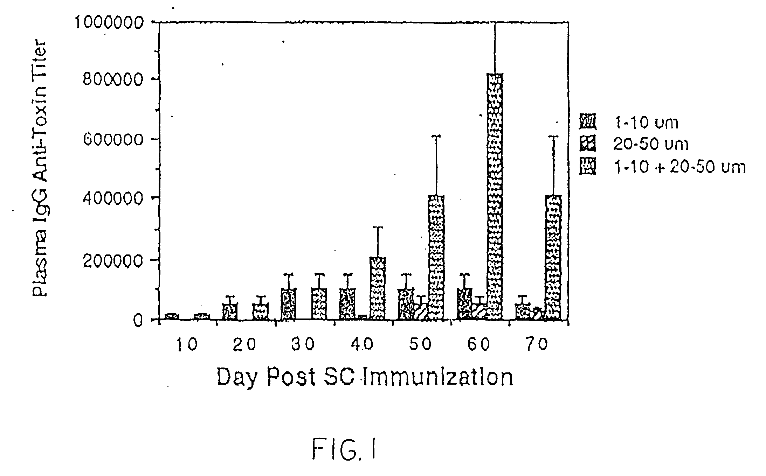

Figure 1 represents the plasma IgA responses in mice determined by endpoint titration.

### DETAILED DESCRIPTION OF THE INVENTION

Illustrations of the methods performing embodiments of the invention follow. These illustrations demonstrate the mucosally-associated lymphoid tissue targeting and programmed delivery of the antigens (trinitrophenyl keyhole limpet hemocyanin and a toxoid vaccine of staphylococcal enterotoxin 8), to mice.

It should be noted, however, that other polymers besides poly(DL-lactide-co-glycolide) may be used. Examples of such polymers include, but are not limited to, poly(glycolide), poly(DL-lactide-co-glycolide), copolyoxalates, polycaprolactone, poly(lactide-co-caprolactone), poly(esteramides), polyorthoesters and poly(8-hydroxybutyric acid), and polyanhydrides.

Also, other immunogenic ingredients nay be used. Examples of such include, but are not limited to, antigens to vaccinate against viral, bacterial, protozoan, fungal diseases such as influenzae, respiratory syncytial, parainfluenza viruses, Hemophilus influenza, Bordetella pertussis, Neisseria gonorrhoeae, Streptococcus pneumoniae and Plasmodium falciparum or other diseases caused by pathogenic microorganisms or antigens to vaccinate against diseases caused by macroorganisms such as helminthic pathogens or antigens to vaccinate against allergies. Additional immunogenic agents which may be used include but are not limited to, immunomodulators, peptides, lymphokines and cytokines.

### I. MICROENCAPSULATION

### A. Preparation of Dye-Loaded Microcapsules

Coumarin, a water-insoluble fluorescent dye, was microencapsulated with polystyrene, which is a nonbiodegradable polymer, to afford fluorescent microcapsules that could be used to follow the penetration of microcapsules into the Peyer's patches. The procedure used to prepare these microcapsules follows:

First, a polymer solution is prepared by dissolving 4.95 g of polystyrene (Type 685D, Dow Chemical Company, Midland, MI) in 29.5 g of methylene chloride (Reagent Grade, Eastman Kodak, Rochester, NY). Next, about 0.05g of coumarin (Polysciences, Inc., Warrington, PA) is added to the polymer solution and allowed to dissolve by stirring the mixture with a magnetic stir bar.

In a separate container, 10 wt% aqueous poly(vinyl alcohol) (PVA) solution, the processing medium, is prepared by dissolving 40 g of PVA (Vinol 2050, Air Products and Chemicals, Allentown, PA) in 360 g of deionized water. After preparing the PVA solution, the solution is saturated by adding 6 g of methylene chloride. Next, the PVA solution is added to a 1-L resin kettle (Ace Glass, Inc., Vineland, NJ) fitted with a truebore stir shaft and a 2.5-in. teflon impeller and stirred at about 380 rpm by a Fisher "stedi speed" motor.

The polystyrene/coumarin mixture is then added to the resin kettle containing the PVA processing media. This is accomplished by pouring the polystyrene/coumarin mixture through a long-stem 7-mm bore funnel which directs the mixture into the resin kettle. A stable oil-in-water emulsion results and is subsequently stirred for about 30 minutes at ambient pressure to afford oil microdroplets of the appropriate size. Then the resin kettle is closed, and the pressure in the resin kettle is gradually reduced to 520 mm Hg by means of a water aspirator connected to a manometer and a bleed valve. The resin kettle contents are stirred at reduced pressure for about 24 hours to allow all of the methylene chloride to evaporate. After all of the methylene chloride has evaporated, the hardened microcapsules are collected by centrifugation and dried for 72 hours in a vacuum chamber maintained at room temperature.

### B. Preparation of Antigen-Loaded Microcapsules

TNP-KLH, a water-soluble antigen, was encapsulated in poly(DL-lactide-co-glycolide), a biocompatible, biodegradable polyester. The procedure used to prepare the microcapsules follows:

First, a polymer solution was prepared by dissolving 0.5g of 50:50 poly(DL-lactide-co-glycolide) in 4.0 g of methylene chloride. Next, 300 microliters of an aqueous solution of TNP-KLH (46 mg TNP-LKH/mL; after dialysis) was added to and homogeneously dispersed in the poly(DL-lactide-co-glycolide) solution by vortexing the mixture with a Vortex-Genie 2 (Scientific Industries, Inc., Bohemia, NY).

In a separate container, an 8 wt% aqueous PVA solution was prepared by dissolving 4.8 g of PVA in 55.2 g of deionized water. After dissolution of the PVA, the PVA solution was added to a 100-mL resin kettle (Kontes Glass, Inc., Vineland, NJ) fitted with a truebore stirrer and a 1.5-in. (3.8 cm) Teflon® turbine impeller. The polymer solution was then added to the PVA processing medium by pouring through a long-stem 7-mm bore funnel. During this addition, the PVA solution was being stirred at about 650 rpm. After the resulting oil-in-water emulsion was stirred in the resin kettle for about 10 minutes, the contents of the resin kettle were transferred to 3.5 L of deionized water contained in a 4-L beaker and being stirred at about 800 rpm with a 2-in. stainless steel impeller. The resultant microcapsules were stirred in the deionized water for about 30 minutes, collected by centrifugation, washed twice with deionized water to remove any residual PVA, and were then collected by freeze drying. The microcapsule products consisted of spherical particles about 1 to 10 micrometers in diameter. Other microcapsules, such as staphylococcal enterotoxin B microcapsules, can be made in a similar manner.

The TNP-KLH content of the antigen-loaded microcapsules, that is, the core loading of the microcapsules, was determined by weighing out 10 mg of antigen-loaded microcapsules in a 12-mL centrifuge tube. Add 3.0 mL of methylene chloride to the tube and vortex to dissolve the poly(DL-lactide-co-qlycolide). Next, add 3.0 mL of deionized water to the tube and vortex vigorously for 1 minute. Centrifuge the contents of the centrifuge tube to separate the organic and aqueous layers. Transfer the aqueous layer to a 10-mL volumetric flask. Repeat the extraction combining the aqueous layers in the volumetric flask. Fill the flask to the mark with deionized water. The amount of TNP-KLH in the flask, and subsequently the amount of TNP-KLH in the microcapsules, is then quantified using a protein assay. The microcapsules contained 0.2% TNP-KLH by weight. The staphylococcal enterotoxin B content of staphylococcal enterotoxin B microcapsules can be quantified in a similar manner.

### Experimental Section:

### II. PENETRATION OF DYE-LOADED MICROCAPSULES INTO THE PEYER'S PATCHES AFTER ORAL ADMINISTRATION

By far the largest mass of tissue with the capacity to function as an inductive site for secretory IgA responses is the Peyer's patches. These discrete nodules of lymphoreticular tissue are located along the entire length of the small intestine and appendix. The targeted delivery of intact antigen directly into this tissue to achieve high local concentration is currently believed to be the most effective means of inducing a disseminated mucosal IgA response. Biodegradable microcapsules represent an ideal vehicle to achieve this targeted vaccination.

### Study - EXAMPLE 1 - Uptake of Polystyrene Microcapsules

The uptake of microcapsules into the gut-associated lymphoreticular tissues and the size restriction of this penetration was investigated by orally administering to mice polystyrene microcapsules, loaded with the fluorescent dye coumarin. Unanesthetized, fasted BALB/c mice were administered 0.5 mL of a 100 mg/mL suspension of various sized fluorescent microcapsules (less than 5 micrometers or 8 to 50 micrometers in diameter) in tap water into the stomach using a feeding needle. At various times after administration (0.5, 1 and 2 hours), the mice were sacrificed and the small intestine excised. One-centimeter sections of gut containing a discrete Peyer's patch were isolated, flushed of lumenal contents, everted and snap frozen. Frozen sections were prepared and examined under a fluorescence microscope to observe the number, location and size of the microcapsules which were taken up into the Peyer's patch from the gut lumen.

Although some trapping of the microcapsules between the villi had prevented their removal during flushing, no penetration into the tissues was observed at any point except the Peyer's patch. At 0.5 hours after oral administration, microcapsules were observed in the Peyer's patch of the proximal, but not the distal, portion of the small intestine. With increasing time the microcapsules were transported by peristaltic movement such that by 2 hours they were throughout the gastrointestinal tract and could be found in the Peyer's patch of the ilium. The endocytosed microcapsules were predominantly located peripherally, away from the apex of the Peyer's patch dome, giving the impression that physical trapping between the dome and adjacent villi during peristalsis had aided in their uptake. Comparison of the efficiency of uptake of the <5 micrometer versus the 8 to 50 micrometer preparations demonstrated that microcapsules >10 micrometers in diameter were not absorbed into the Peyer's patches while microcapsules of 1 to 10 micrometers in diameter were rapidly and selectively taken up. This suggested that microcapsules composed of biodegradable wall materials would serve as an effective means for the targeted delivery of antigens to the lymphoreticular tissues for the induction of immunity at mucosal surfaces.

### Study - EXAMPLE 2 - 85:15 Poly(DL-lactide-co-glycolide) Microcapsules

### 1. Uptake of Biocompatible and Biodegradable Microcapsules into the Peyer's Patches

Groups of mice were administered biodegradable microcapsules containing the fluorescent dye coumarin-6 as a suspension in tap water via a gastric tube. The microcapsule wall material chosen for these studies consisted of 85:15 poly(DL-lactide-co-glycolide) due to its ability to resist significant bioerosion for a period of six weeks. At various times from 1 to 35 days after administration, three representative Peyer's patches, the major mesenteric lymph nodes and the spleens from individual mice were removed, processed and serial frozen sections prepared.

When viewed with a fluorescence microscope using appropriate excitation and barrier filters the coumarin exhibited a deep green fluorescence which allowed the visual detection of microcapsules substantially less than 1 micrometer in diameter. All sections were viewed in order that the total number of microcapsules within each tissue or organ could be quantified. The size of each internalized microcapsule was determined using a calibrated eyepiece micrometer and its location within the tissue or organ was noted.

Internalized microcapsules of various sizes were observed in the Peyer's patches at 24 hours post oral administration and at all time points tested out to 35 days, as shown in Table 1. At no time were microcapsules of any size observed to penetrate into the tissue of the gut at any point other than the Peyer's patches. The total number of microcapsules within the Peyer's patches increased through Day 4 and then decreased over the following 31 days to approximately 15% of the peak number.

This is consistent with the observation that free microcapsules could be observed on the surface of the gut villi at the 1, 2 and 4 day time points. It is of interest that approximately 10 hours following oral administration of the microcapsule suspension the coumarin-loaded microcapsules were frankly observable in the passed feces. This clearance was followed with the aid of an ultraviolet light source and by 24 hours the vast majority of the ingested microcapsules had been passed. Thus, the continued uptake of microcapsules into the Peyer's patches observed at 2 and 4 days must be attributed to the minor fraction of the input dose which became entrapped within mucus between the gut villi. In addition, the efficiency of uptake for the entrapped microcapsules must be several orders of magnitude greater than that of the microcapsules present in the gut lumen, but above the mucus layer. These observations are important when these data are extrapolated to man; the tremendously larger mass of Peyer's patch tissue and the greatly increased transit time for the passage of material through the human small intestine relative to the mouse suggests that the efficiency of microcapsule uptake into the human Peyer's patches will be much higher.

Microcapsules of various sizes were observed within the Peyer's patches at all time points tested as shown in Table 1. At the 1, 2 and 4 day time points the proportion of <2 micrometers (45-47%), 2-5 micrometers (31-35%) and >5 micrometers (18-23%) microcapsules remained relatively constant. Evident at 7 days, and even more so at later time points, was a shift in the size distribution such that the small (<2 micrometers) and medium (2-5 micrometers) microcapsules ceased to ,predominate and the large (>5 micrometers) microcapsules became the numerically greatest species observed. This shift was concurrent with the decrease in total microcapsule numbers in the Peyer's patches observed on and after Day 7. These results are consistent with the preferential migration of the small and medium sizes of microcapsules from the Peyer's patches while the large (>5 micrometers) microcapsules are preferentially retained.

Consistent with the preferential migration of the small and medium microcapsules out of the Peyer's _patches are the data pertaining to the location of microcapsules within the architecture of the Peyer's patches. When a microcapsule was observed within the Peyer's patch, it was noted to be either relatively close to the dome epithelium where it entered the Peyer's patch (within 200 micrometers) or deeper within the lymphoid tissue (≥200 micrometers from the closest identifiable dome epithelium) (Table 1). Microcapsules observed deep within the Peyer's patch tissue were almost exclusively of small and medium diameter. At 1 day post-administration, 92% of the microcapsules were located close to the dome epithelium. The proportion of deeply located microcapsules increased through Day 4 to 24% of the total, and thereafter decreased with time to approximately 2% at Day 14 and later. Thus, the small and medium microcapsules migrate through and out of the Peyer's patches, while the large (>5 micrometers) microcapsules remain within the dome region for an extended period of time.

### 2. Microcapsule Migration to the Mesenteric Lymph Nodes and Spleen

A small number of microcapsules were observed in the mesenteric lymph nodes at 1 day post-administration, and the numbers progressively increased through Day 7, as shown in Table 2. After Day 7, the numbers decreased but were still detectable on Day 35. The size distribution clearly showed that microcapsules >5 micrometers in diameter did not enter this tissue, and the higher proportion of small (<2 micrometers) relative to medium (2-5 micrometers) microcapsules at the earlier time points indicated that the smaller diameter microcapsules migrate to this tissue with greatest efficiency. In addition, at the earlier time points, the majority of the microcapsules were located just under the capsule in the subcapsular sinus. Later time points showed a shift in the distribution to deep within the lymph node structure, and by day 14, 90% of the microcapsules were located within the cortex and medullary regions. The observation that the microcapsules are first detected in or near the subcapsular sinus is consistent with their entry into this tissue via the lymphatics which drain the Peyer's patches. A progressive increase in the proportion of the microcapsules located deep in this tissue, clearly discernable at Day 4, followed by a progressive drop in the total numbers on Day 14 and later, suggests that the microcapsules progress through this tissue and extravasate through the efferent lymphatic drainage.(*)

Similar examination of the spleen showed that no microcapsules were detectable until Day 4 post-administration. Peak numbers of microcapsules were not observed in this organ until Day 14. As in the case of the mesenteric lymph nodes, no microcapsules of >5 micrometers in diameter were observed. At all time points, the microcapsules were observed deep in this organ within the cortex. It should be noted that the peak number of microcapsules was observed in the spleen at a time when the majority of the microcapsules present in the mesenteric lymph nodes was deeply located and their total numbers falling. These data are consistent with the known pattern of lymph drainage from the Peyer's patches to the mesenteric lymph nodes and from the mesenteric lymph nodes to the bloodstream via the thoracic duct. Thus, it appears that the microcapsules present in the spleen have traversed the Peyer's patches and mesenteric lymph nodes and have entered the spleen via the blood circulation.

In additional experiments, tissue sections from Peyer's patches, mesenteric lymph node and spleen which contained absorbed 85:15 DL-PLG microcapsules were examined by histochemical and immunohistochemical techniques. Among other observations, these studies clearly showed that the microcapsules which were absorbed into the Peyer's patches were present within macrophage-like cells which were stained by periodic acid Schiff's reagent (PAS) for intracellular carbohydrate, most probably glycogen, and for major histocompatibility complex (MHC) class II antigen. Further, the microcapsules observed in the mesenteric lymph nodes and in the spleen were universally found to have been carried there within these PAS and MHC class II positive cells. Thus, the antigen containing microcapsules have been internalized by antigen-presenting accessory cells (APC) in the Peyer's patches, and these APC have disseminated the antigen-microcapsules to other lymphoid tissues.

These data indicate that the quality of the immune response induced by orally administering a microencapsulated vaccine can be controlled by the size of the particles. Microcapsules <5 micrometers in diameter extravasate from the Peyer's patches within APC and release the antigen in lymphoid tissues which are inductive sites for systemic immune responses. In contrast, the microcapsules 5 to 10 micrometers in diameter remain in the Peyer's patches, also within APC, for extended time and release the antigen into this sIgA inductive site.

### Study - EXAMPLE 3 - Comparison of the Uptake of Microcapsules of 10 Compositions by the Peyer's Patches

Experiments were performed to identify microcapsule polymeric excipients that would be useful for a practical controlled release delivery system and which would possess the physicalchemical properties which would allow for targeted absorption of microcapsules into the mucosally-associated lymphoid tissues. In regard to the latter consideration, research has shown that hydrophobic particles are more readily phagocytized by the cells of the reticuloendothelial system. Therefore, the absorption into the Peyer's patches of 1- to 10-micrometer microcapsules of 10 different polymers which exhibit some range with respect to hydrophobicity was examined. The wall materials chosen for these studies consisted of polymers that varied in water uptake, biodegradation, and hydrophobicity. These polymers included polystyrene, poly(L-lactide), poly(DL-lactide), 50:50 poly(DL-lactide-co-glycolide), 85:15 poly(DL-lactide-co-glycolide), poly(hydroxybutyric acid), poly(methyl methacrylate), ethyl cellulose, cellulose acetate hydrogen phthalate, and cellulose triacetate. Microcapsules, prepared from 7 of the 10 excipients, were absorbed and were predominantly present in the dome region of the Peyer's patches 48 hours after oral administration of a suspension containing 20 mg of microcapsules, as shown in Table 3. None of the microspheres were seen to penetrate into tissues other than the Peyer's patches. With one exception, ethyl cellulose, the efficiency of absorption was found to correlate with the relative hydrophobicity of the excipient. Up to 1,500 microcapsules were observed in the 3 representative Payer's patches of the mice administered the most hydrophobic group of compounds [poly(styrene), poly(methyl methacrylate), poly(hydroxybutyrate)], while 200 to 1,000 microcapsules were observed with the relatively less hydrophobic polyesters [poly(L-lactide), poly(DL-lactide), 85:15 poly(DL-lactide-co-glycolide), 50:50 poly(OL-lactide-co-glycolide)]. As a class, the cellulosics were not absorbed..

It has been found that the physicalchemical characteristics of the microcapsules regulate the targeting of the microcapsules through the efficiency of their absorption from the gut lumen by the Peyer's patches, and that this is a surface phenomenon. Therefore, alterations in the surface characteristics of the microcapsules, in the form of chemical modifications of the polymer or in the form of coatings, can be used to regulate the efficiency with which the microcapsules target the delivery of bioactive agents to mucosally-associated lymphoid tissues and to APC. Examples of coatings which may be employed but are not limited to, chemicals, polymers, antibodies, bioadhesives, proteins, peptides, carbohydrates, lectins and the like of both natural and man made origin.

### Collection of Biological Fluids.

1. Plasma. Blood was collected in calibrated capillary pipettes following puncture of the retro-orbital plexus. Following clot formation, the serum was collected, centrifuged to remove red cells and platelates, heat-inactivated, and stored at -70°C until assayed.
2. Intestinal Secretions. Mice were administered four doses (0.5 mL) of lavage solution (25 mM Nacl, 40 mM Na₂SO₄, 10 mM KCl, 20 mM NaHCO₃, and 48.5 mM poly(ethylene glycol), osmolarity of 530 mosM) at 15-minute intervals (Elson, C.O., Ealding, W. and Lefkowitz, J. A lavage technique allowing repeated measurement of IgA antibody on mouse intestinal secretions. J. Immunol. Meth. 67:101; 1984). Fifteen minutes after the last dose of lavage solution, the mice were anesthetized and after an additional 15 minutes they were administered 0.1 mg pilocarpine by ip injection. Over the next 10 to 20 minutes, a discharge of intestinal contents was stimulated. This was collected into a petri dish containing 3 mL of a solution of 0.1 mg/mL soybean trypsin inhibitor (Sigma, St. Louis, MO) in 50 mM EDTA, vortexed vigorously and centrifuged to remove suspended matter. The supernatant was transferred to a round-bottom, polycarbonate centrifuge tube and 30 microgliters of 20 millimolar phenylmethylsulfonyl fluoride (PMSF, Sigma) was added prior to clarification by high-speed centrifugation (27,000 x g, 20 minutes, 4°C). After clarification, 20 microliters each of PMSF and 1% sodium azide were added and the solution made 10% in FCS to provide an alternate substrate for any remaining proteases.
3. Saliva. Concurrent with the intestinal discharge, a large volume of saliva is secreted and 0.25 mL was collected into a pasteur pipette by capillary action. Twenty microliters each of trypsin inhibitor, PMSF, sodium azide and FCS was added prior to clarification.
4. Bronchial-Alveolar Wash Fluids. Bronchial-alveolar wash fluids were obtained by lavaging the lungs with 1.0 mL of PBS. An animal feeding needle was inserted intratracheally and fixed in place by tying with suture material. The PBS was inserted and withdrawn 5 times to obtain washings, to which were added 20 microliters each of trypsin inhibitor, PMSF, sodium azide, and FCS prior to clarification by centrifugation.
5. Immunochemical Reagents. Solid-phase absorbed and affinity-purified polyclonal goat IgG antibodies specific for murine IgM, IgG and IgA were obtained commercially (Southern Biotechnology Associates, Birmingham, AL). Their specificity in radioimmunoassays was tested through their ability to bind appropriate purified monoclonal antibodies and myeloma proteins.
6. Solid-Phase Radioimmunoassays. Purified antibodies were labeled with carrier-free Na ¹²⁵l (Amersham) using the chloramine T method (Hunter, W.M. Radioimmunoassay. In: Handbook of Experimental Immunology, M. Weir (editor). Blackwell Scientific Publishing, Oxford, p. 14.1; 1978). Immulon Removawell assay strips (Dynatech) were coated with TNP conjugated bovine serum albumin (BSA) or staphylococcal enterotoxin B at 1 microgram/mL in BBS overnight at 4°C. Control strips were left uncoated but all strips were blocked for 2 hours at room temperature with 1% BSA in BBS, which was used as the diluent for all samples and ¹²⁵l-labeled reagents. Samples of biologic fluids were appropriately diluted, added to washed triplicate replicate wells, and incubated 6 hours at room temperature. After washing, 100,000 cpm of ¹²⁵l-labeled isotype-specific anti-immunoglobulin was added to each well and incubated overnight at 4°C. Following the removal of unbound 1251-antibodies by washing, the wells were counted in a Gamma 5500 spectrometer (Beckman Instruments, Inc., San Ramon, CA). In the case of the assays for TNP specific antibodies, calibrations were made using serial twofold dilutions of a standard serum (Miles Scientific, Naperville, IL) containing known amounts of immunoglobulins, on wells coated with 1 microgram/well isotype-specific antibodies. Calibration curves and interpolation of unknowns was obtained by computer, using "Logit-log" or "Four Parameter Logistic" BASIC Technology Center (Vanderbilt Medical Center, Nashville, TN). In the case of antibodies specific to staphylococcal enterotoxin B, the results are presented as the reciprocal serum dilution producing a signal >3-fold that of the group-matched prebleed at the same dilution (end-point titration).

Research in our laboratories has shown that microencapsulation results in a profoundly heightened immune response to the incorporated antigen or vaccine in numerous experimental systems. An example is provided by the direct comparison of the level and isotype distribution of the circulating antibody response to Staphylococcal enterotoxin B, the causative agent of Staphylococcal food poisoning, following immunization with either soluble or microencapsulated enterotoxoid.

### 2. Mechanism of the Adjuvant Effect Imparted by Microencapsulation.

### EXAMPLE 1 - The Adjuvant Effect Imparted by Microencapsulation is Not the Result of Adjuvant Activity Intrinsic to the Polymer.

When considering the mechanism through which 1-10 micrometer DL-PLG microspheres mediate a potentiated humoral immune response to the encapsulated antigen, three mechanisms must be considered as possibilities. First, the long term chronic release (depot), as compared to a bolus dose of nonencapsulated antigen, may play a role in immune enhancement. Second, our experiments have shown that microspheres in this size range are readily phagocytized by antigen processing and presenting cells. Therefore, targeted delivery of a comparatively large dose of nondegraded antigen directly to the cells responsible for the initiation of immune responses to T cell-dependent antigens must also be considered. Third, the microcapsules may possess intrinsic immunopotentiating activity through their ability to activate cells of the immune system in a manner analogous to adjuvants such as bacterial lipopolysaccharide or muranyl-di-peptide. Immunopotentiation by this latter mechanism has the characteristic that it is expressed when the adjuvant is administered concurrently with the antigen.

In order to test whether microspheres posses any inate adjuvancy which is mediated through the ability of these particles to nonspecifically activate the immune system, the antibody response to 100 micrograms of microencapsulated enterotoxoid was compared to that induced following the administration of an equal dose of enterotoxoid mixed with placebo microspheres containing no antigen. The various antigen forms were administered by IP injections into groups of 10 BALB/c mice and the plasma IgM and IgG enterotoxin-specific antibody responses determined by end-point titration RIAs, as shown in Table 4.

The plasma antibody response to a bolus injection of the optimal dose of soluble enterotoxoid (25 micrograms) was characteristically poor and consisted of a peak IgM titer of 800 on day 10 and a peak IgG titer of 800 on day 20. Administration of an equal dose of microencapsulated enterotoxoid induced a strong response in both the IgM and IgG isotypes which was still increasing on day 30 after immunization. Coadministration of soluble enterotoxoid and a dose of placebo microspheres equal in weight, size and composition to those used to administer encapsulated antigen did not induce a plasma anti-toxin response which was significantly higher than that induced by soluble antigen alone. This result was not changed by the administration of the soluble antigen 1 day before or 1, 2 or 5 days after the placebo microspheres. Thus, these data indicate that the immunopotentiation expressed when antigen is administered within 1-10 micrometer DL-PLG microspheres is not a function of the ability of the microspheres to intrinsically activate the immune system. Rather, the data are consistent with either a depot effect, targeted delivery of the antigen to antigen-presenting accessory cells, or a combination of these two mechanisms.

### EXAMPLE 2 - Retarding the Antigen Release Rate from 1-10 Micrometer Microcapsules Increases the Level of the Antibody Response and Delays the Time of the Peak Response.

Four enterotoxoid containing microcapsule preparations with a variety of antigen release rates were compared for their ability to induce a plasma anti-toxin response following IP injection. The rate of antigen release by the microcapsules used in this study is a function of two mechanisms; diffusion through pores in the wall matrix and hydrolysis (bioerosion) of the wall matrix. Batches #605-026-1 and #514-140-1 have varying initial rates of release through pores, followed by a second stage of release which is a function of their degradation through hydrolysis. In contrast, Batches #697-143-2 and #928-060-00 have been manufactured with a tight uniform matrix of wall material which has little release through pores and their release is essentially a function of the rate at which the wall materials are hydrolyzed. However, these latter two lots differ in the ratio of lactide to glycolide composing the microcapsules, and the greater resistance of the 85:15 DL-PLG to hydrolysis results in a slower rate of enterotoxoid release.

The immune response induced by Batch #605-026-1 (60% release at 48 hours) reached a peak IgG titer of 6,400 on day 20 (Table 5). Batch #514-140-1 (30% release at 48 hours) stimulated IgG antibodies which also peaked on day 20, but which were present in higher concentration both on days 20 and 30.)

Immunization with Batch #697-143-2 (10% release at 48 hours) resulted in peak IgG antibody levels on days 30 and 45 which were substantially higher (102,400) than those induced by either lot with early release. Further delaying the rate of antigen release through the use of an 85:15 ratio of lactide to glycolide, Batch #928-060-00 (0% release at 48 hours) delayed the peak antibody levels until *days 45* and 60, but no further increase in immunopotentiation was observed.

These results are consistent with a delayed and sustained release of antigen stimulating a higher antibody response. However, certain aspects of the pattern of responses induced by these various microspheres indicate that a depot effect is not the only mechanism of immunopotentiation. The faster the initial release, the lower the peak antibody titer. These results are consistent with a model in which the antigen released within the first 48 hours via diffusion through pores is no more effective than the administration of soluble antigen. Significant delay in the onset of release to allow time for phagocytosis of the microspheres by macrophages allows for the effective processing and presentation of the antigen, and the height of the resulting response is governed by the amount of antigen delivered into the presenting cells. However, delay of antigen release beyond the point where all the antigen is delivered into the presenting cells does not result in further potentiation of the response, it only delays the peak level.

### 2. Pulsatile Release of Vaccines from Microcapsules for Programmed Boosting Following a Single Injection

When one receives any of a number of vaccines by injection, two to three or more administrations of the vaccine are required to elicit a good immune response. Typically, the first injection is given to afford a primary response, the second injection is given to afford a secondary response, and a third injection is given to afford a tertiary response. Multiple injections are needed because repeated interaction of the antigen with immune system cells is required to stimulate a strong immunological response. After receiving the first injection of vaccine, a patient, therefore, must return to the physician on several occasions to receive the second, third, and subsequent injections to acquire protection. Often patients never return to the physician to get the subsequent injections.

The vaccine formulation that is injected into a patient may consist of an antigen in association with an adjuvant. For instance, an antigen can be bound to alum. During the first injection, the use of the antigen/adjuvant combination is important in that the adjuvant aids in the stimulation of an immune response. During the second and third injections, the administration of the antigen improves the immune response of the body to the antigen. The second and third administrations or subsequent administrations, however, do not necessarily require an adjuvant.

Alza Corporation has described methods for the continuous release of an antigen and an immunopotentiator (adjuvant) to stimulate an immune response (U.S. Patent No. 4,455,142). This invention differs from the Alza patent in at least two important manners. First, no immunopotentiator is required to increase the immune response, and second, the antigen is not continuously released from the delivery system.

The present invention concerns the formulation of vaccine (antigen) into microcapsules (or microspheres) whereby the antigen is encapsulated in biodegradable polymers, such as poly(DL-lactide-co-glycolide). More specifically, different vaccine microcapsules are fabricated and then mixed together such that a single injection of the vaccine capsule mixture improves the primary immune response and then delivers antigen in a pulsatile fashion at later time points to afford secondary, tertiary, and subsequent responses.

The mixture of microcapsules consists of small and large microcapsules. The small microcapsules, less than 10 microns, preferably less than 5 micrometers, or more preferably 1 to 5 micrometers, potentiate the primary response (without the need of an adjuvant) because the small microcapsules are efficiently recognized and taken up by macrophages. The microcapsules inside of the macrophages then release the antigen which is subsequently processed and presented on the surface of the macrophage to give the primary response. The larger microcapsules, greater than 5 micrometers, preferably greater than 10 microns, but not so large that they cannot be administered for instance by injection, preferably less than 250 micrometers, are made with different polymers so that as they biodegrade at different rates, they release antigen in a pulsatile fashion.

Using the present invention, the composition of the antigen microcapsules for the primary response is basically the same as the composition of the antigen microcapsules used for the secondary, tertiary, and subsequent responses. That is, the antigen is encapsulated with the same class of biodegradable polymers. The size and pulsatile release properties of the antigen microcapsules then maximizes the immune response to the antigen.

The preferred biodegradable polymers are those whose biodegradation rates can be varied merely by altering their monomer ratio, for example, poly(DL-lactide-co-glycolide), so that antigen microcapsules used for the secondary response will biodegrade faster than antigen microcapsules used for subsequent responses, affording pulsatile release of the antigen.

In summary, by controlling the size of the microcapsules of basically the same composition, one can maximize the immune response to an antigen. Also important is having small microcapsules (microcapsules less than 10 micrometers, preferably less than 5 micrometers, most preferably 1 to 5 micrometers) in the mixture of antigen microcapsules to maximize the primary response. The use of an immune enhancing delivery system, such as small microcapsules, becomes even more important when one attempts to elicit an immune response to less immunogenic compounds such as killed vaccines, subunit vaccines, low-molecular-weight vaccines such as peptides, and the like.

### EXAMPLE 1 - Coadministration of Free and Microencapsulated Vaccine.

A Japanese Encephalitis virus vaccine (Biken) was studied. The virus used is a product of the Research Foundation for Microbial Disease of Osaka University, Suita, Osaka, Japan. The manufacturer recommends a three dose immunization series consisting of two doses of vaccine administered one to two weeks apart followed by administration of a third dose of vaccine one month after the initial immunization series. We have compared the antiviral immune responses of mice immunized with a standard three dose schedule of JE vaccine to the antiviral response of mice immunized with a single administration of JE vaccine consisting of one part unencapsulated vaccine and two parts encapsulated vaccine. The JE microcapsules were >10 micrometers. The results of immunizing mice with JE vaccine by these two methods were compared by measuring the serum antibody titers against JE vaccine detected through an ELISA assay. The ELISA assay measures the presence of serum antibodies with specificity of JE vaccine components, however, it does not measure the level of virus neutralizing antibody present in the serum. The virus neutralizing antibody activity was therefore measured by virus cytopathic effect (CPE) inhibition assays and virus plaque reduction assays. The results of those assays are presented here.

Four experimental groups consisting of (1) untreated control mice which receive no immunization; (2) mice which received 3.0 mg of JE vaccine (unencapsulated) on Day 0; (3) nice which received 3.0 mg of JE vaccine (unencapsulated) on Days 0, 14 and 42 (standard schedule) and (4) mice which received 3.0 mg of JE vaccine (unencapsulated) and 3.0 mg of JE vaccine (encapsulated) on day 0 were studied. The untreated controls provide background virus neutralization titers against which immunized animals can be compared. The animals receiving a single 3.0 mg dose of JE vaccine on Day 0 provide background neutralization titers against which animals receiving unencapsulated vaccine in conjunction with encapsulated vaccine can be compared. This comparison provides evidence that the administration of encapsulated vaccine augments the immunization potential of a single 3.0 mg dose of unencapsulated vaccine. The animals receiving 3 doses of unencapsulated vaccine provide controls against which the encapsulated vaccine group can be compared so as to document the ability of a single injection consisting of both nonencapsulated and encapsulated vaccine to produce antiviral activity comparable to a standard three dose immunization schedule.

Serum samples collected on Days 21, 49 and 77 from ten animals in each experimental group were tested for their ability to inhibit the cytopathic effects induced by a standard challenge (100 TCID₅₀) of JE virus. The results of the CPE inhibition assays, expressed as the highest serum dilution capable of inhibiting 50% of the viral CPE, are presented in Table 6. As is shown, the untreated control animals (Group 1) had no significant serum virus neutralizing activity at any point tested. Of the ten animals receiving a single 3.0 mg dose of JE vaccine on Day 0 (Group 2), one did not develop any detectable virus neutralizing antibody. Of the remaining nine mice, the highest titer achieved was 254 which occurred on Day 49. The geometric mean antiviral titer for this experimental group peaked on Day 49. Of the ten animals receiving a standard schedule of three vaccine doses (Group 3), eight had a decrease in antibody activity from Day 49 to Day 77. The geometric mean titer for this group decreased by greater than 50% from Day 40 to Day 77. All ten animals receiving encapsulated JE vaccine (Group 4) developed serum antiviral activity. The geometric mean titer for this group increased from Day 21 to Day 77. The average titer occurring on Day 49 in this group was significantly lower than that occurring in the 3 vaccine dose group (Group 3) (p = 0.006); however, the titer continued to increase from Day 49 to Day 77 which is in contrast to the 3 vaccine dose group. There was no significant difference in the average titer for these two groups in the Day 77 samples (p = .75) indicating that the encapsulated vaccine group achieved comparable serum antiviral titers at Day 77. Unlike the 3 vaccine dose group (Group 3), the animals receiving encapsulated vaccine (Group 4) continued to demonstrate increases in serum virus neutralizing activity throughout the timepoints examined. In contrast to the standard vaccine treatment group, mice receiving encapsulated JE vaccine had a two-fold increase in the average serum neutralizing titer from Day 49 to Day 77. The Day 21 average antiviral titer from mice receiving microencapsulated vaccine was not significantly different from the Day 21 average titer of mice receiving a single dose of JE vaccine on Day 0 (p = .12); however, the day 49 and Day 77 average titers were significantly different for the two groups (p = .03 and p = .03, respectively). These data indicate that serum virus neutralizing titers similar to those produced by standard vaccine administration can be achieved by administering a single dose of encapsulated JE vaccine. Although the antiviral titers achieved with the excipient formulation used in this study did not increase as rapidly as those achieved with the standard vaccine, the serum neutralizing antibody activity did reach titers which are comparable to those achieved with the standard three dose vaccine schedule.

To further corroborate these findings, pooled samples produced by mixing equal volumes of each serum sample were prepared for each experimental group. These samples were submitted to an independent laboratory for determination of antiviral activity. The samples were tested by plaque reduction assay against a standard challenge of JE virus. The results of these assays, presented in Table 7, substantiate the findings described above. Although the animals receiving encapsulated vaccine did not reach peak titers as rapidly as did the standard vaccine group, the encapsulated vaccine did induce comparable virus neutralizing antibody activity. Furthermore, the encapsulated vaccine maintained a higher antiviral titer over a longer period of time than did the standard vaccine. These results further support the conclusion that a single administration of microencapsulated vaccine can produce results comparable to those achieved with a three dose schedule of standard vaccine.

### EXAMPLE 2 - Coadministration of <10 Micrometer and >10 Micrometer vaccine Microcapsules.

One advantage of the copolymer microcapsule delivery system is the ability to control the time and/or rate at which the incorporated material is released. In the case of vaccines this allows for scheduling of the antigen release in such a manner as to maximize the antibody response following a single administration. Among the possible release profiles which would be expected to improve the antibody response to a vaccine is a pulsed release (analogous to conventional booster immunizations).

The possibility of using a pulsed release profile was investigated by subcutaneously administering 100 micrograms of enterotoxoid to groups of mice either in 1-10 micrometer (50:50 DL-PLG; 1.51 wt% enterotoxoid), 20-125 µm (50:50 DL-PLG; 0.64 wt% enterotoxoid) or in a mixture of 1-10 micrometer and 20-125 micrometer microcapsules in which equal parts of the enterotoxoid were contained within each size range. The groups of mice were bled at 10 day intervals and the plasma IgG responses were determined by endpoint titration in isotype-specific immunoradiometric assays employing solid-phase absorbed enterotoxin (Figure 1). Following the administration of the 1-10 micrometer enterotoxoid microcapsules the plasma IgG response was detected on day 10, rose to a maximal titer of 102,400 on days 30 and 40, and decreased through day 60 to 25,600. In contrast, the response to the toxoid administered in 20-125 micrometer microcapsules was delayed until day 30, and thereafter increased to a titer of 51,200 on days 50 and 60. The concomitant administration of equal parts of the toxoid in 1-10 and 20-125 micrometer microcapsules produced an IgG response which was for the first 30 days essentially the same as that stimulated by the 1-10 micrometer microcapsules administered alone. However, beginning on day 40 the response measured in the mice concurrently receiving the 1-10 plus 20-125 micrometer microcapsules steadily increased to a titer of 619,200 on day 60, a level which was far more than the additive amount of the responses induced by the two size ranges administered singly.

The antibody response obtained through the coadministration of 1-10 and 20-125 micrometer enterotoxoid-containing microcapsules is consistent with a two phase (pulsed) release of the antigen. The first pulse results from the rapid ingestion and accelerated degradation of the 1-10 micrometer particles by tissue histiocytes, which results in a potentiated primary immune response due to the efficient loading of high concentrations of the antigen into these accessory cells, and most probably their activation. The second phase of antigen release is due to the biodegradation of the 20-125 micrometer microcapsules, which are too large to be ingested by phagocytic cells. This second pulse of antigen is released into a primed host and stimulates an anamnestic immune response. Thus, using the 50:50 DL-PLG copolymer, a single injection vaccine delivery system can be constructed which potentiates antibody responses (1-10 micrometer microcapsules), and which can deliver a timed and long lasting secondary booster immunization (20-125 micrometer microcapsules). In addition, through alteration of the ratio of the copolymers, it is possible to prepare formulations which release even later, in order to provide tertiary or even quaternary boostings without the need for additional injections.

Therefore, there exist a number of possible approaches to vaccination by the injectable microcapsules of the present invention. Among these include multiple injections of small microcapsules, preferably 1 to 5 micrometers, that will be engulfed by macrophages and obviate the need for immunopotentiators, as well as mixtures of free antigen for a primary response in combination with microcapsulated antigen in the form of microcapsules having a diameter of 10 micrometers or greater that release the antigen pulsatile to potentiate secondary and tertiary responses and provide immunization with a single administration. Also, a combination of small microcapsules for a primary response and larger microcapsules for secondary and later responses may be used, thereby obviating the need for both immunopotentiators and multiple injections.

### C. Vaccine Microcapsules Administered Intratracheally.

### EXAMPLE 1 - Intratracheally Administered Microcapsules containing SEB Toxoid Induce concurrent Circulating and Mucosal Anti-Toxin Antibodies.

Folliculi lymphatic aggregati similar to the Peyer's patches of the gastrointestinal tract are present in the mucosally-associated lymphoid tissues found at other anatomical locations, such as the respiratory tract. Their function is similar to that of the Peyer's patches in that they absorb materials from the lumen of the lungs and are inductive sites for antibody responses which are characterized by a high proportion of sIgA. The feasibility of immunization through the bronchial-associated lymphoid tissue was investigated. Groups of mice were administered 50 microliters of PBS containing 50 micrograms of SEB toxoid in either microencapsulated or nonencapsulated form directly into the trachea. On days 10, 20, 30 and 40 following the immunization, samples of plasma, "saliva, gut washings and bronchial-alveolar washings were collected.

Assay of the plasma samples for anti-toxin specific antibodies revealed that the administration of free SE8 toxoid did not result in the induction of a detectable antibody response in any isotype (Table 8). In contrast, intratracheal instillation of an equal dose of microencapsulated SEB vaccine elicited toxin specific antibodies of all isotypes. This response reached maximal levels on Day 30 and was maintained through day 40 with IgM, IgG and IgA titers of 400, 51,300 and 400, respectively.

Similar to the responses observed in the plasma, toxin-specific antibodies in the bronchial-alveolar washings were induced by the microencapsulated toxoid, but not by the nonencapsulated vaccine (Table 9). The kinetics of the appearance of the anti-toxin antibodies in the bronchial secretions was delayed somewhat as compared to the plasma response in that the Day 20 response was only detected in the IgG isotype and was low in comparison to the plateau levels eventually obtained. However, maximal titers of IgG and IgA anti-toxin antibodies (1,280 and 320, respectively) were attained by Day 30 and were maintained through Day 40. No IgM class antibodies were detected in the bronchial-alveolar washings using this immunization method, a result consistent with the absence of IgM secreting plasma cells in the lungs and the inability of this large antibody molecule to transudate from the serum past the approximately 200,000 molecular weight cut off imposed by the capillary-alveolar membrane.

These data demonstrate that microencapsulation allowed an immune response to take place against the antigen SEB toxoid following administration into the respiratory tract while the nonencapsulated antigen was ineffective. This response was observed both in the circulation and in the secretions bathing the respiratory tract. It should be noted that this immunization method was effective at inducing the appearance of IgA class antibodies. This antibody is presumably the product of local synthesis in the upper respiratory tract, an area which is not protected by the IgG class antibodies which enter the lower lungs from the blood circulation. Thus, intratracheal immunization with microencapsulated antigens, through the inhalation of aerosols, will be an effective means of inducing antibodies which protect the upper respiratory tract.

### D. Vaccine Microcapsules Administered by Mixed Immunization Routes.

In both man and animals, it has been shown that systemic immunization coupled with mucosal presentation of antigen is more effective than any other combination in promoting mucosal immune responses (Pierce, N.F. and Gowans, J.L. Cellular kinetics of the intestinal immune response to cholera toxoid in rats. J. Exp. Med. 142:1550; 1975). Three groups of mice were primed by IP immunization with 100 micrograms of microencapsulated SEB toxoid and 30 days later were challenged with 100 micrograms of microencapsulated SEB toxoid by either the IP, oral or IT routes. This was done to directly determine if a mixed immunization protocol utilizing microencapsulated antigen was advantageous with respect to the levels of sIgA induced.

Twenty days following the microencapsulated booster immunizations, samples of plasma, gut washings and bronchial-alveolar washings were obtained and the levels and isotype distribution of the anti-SEB toxin antibodies were determined in endpoint titration radioimmunoassays (Table 10). The IP boosting of IP primed mice led to the appearance of high levels of IgG anti-toxin antibodies in the samples of plasma and secretions, but was completely ineffective at the induction of detectable IgA antibodies in any fluid tested. In contrast, secondary immunization with microencapsulated SEB toxoid by either the oral or IT routes efficiently boosted the levels of specific IgG antibodies in the plasma (pre-secondary immunization titer in each group was 51,200) and also induced the appearance of significant levels of sIgA antibodies in the gut and bronchial-alveolar washings. Oral boosting of IP primed mice induced sIgA anti-SEB toxin antibodies to be secreted into the gut secretions at levels which were comparable with those requiring three spaced oral immunizations (Table 10 as compared to Comp. Table 11). Intratracheal boosting of previously IP immunized mice was particularly effective in the induction of a disseminated mucosal response and elicited the appearance of high concurrent levels of IgG and sIgA antibodies in both the samples of bronchial-alveolar and gut secretions.

These results are particularly important with respect to immunization against numerous infectious agents which exert their pathophysiologic effects through acute infections localized to the respiratory tract. Antibodies present within the respiratory tract originate from two different sources. Secretory IgA predominates in the mucus which bathes the nasopharynx and bronchial tree (Soutar, C.A. Distribution of plasma cells and other cells containing immunoglobulin in the respiratory tract of normal man and class of immunoglobulin contained therein. Thorax 31:58; 1976 and Kaltreider, H.B. and Chan, M.K.L. The class-specified immunoglobulin composition of fluids obtained from various levels of canine respiratory tract. J. Immunol. 116:423; 1976) and is the product of local plasma cells which are located in the lamina propria of the upper respiratory tract. In contrast to the nasopharynx and bronchial tree, the bronchioli and alveoli predominantly contain IgG which is passively-derived from the blood circulation via transhudation (Reynolds, H.Y. and Newball, H.H. Analysis of proteins and respiratory cells obtained from human lungs by bronchial lavage. J. Lab. Clin. Med. 84:559; 1974). Thus, effective protection of the lungs requires both circulating IgG and mucosal sIgA antibodies.

These data indicate that mixed route immunization protocols utilizing microencapsulated antigens will prove the most efficient in the induction of concurrent circulating and mucosal antibody responses. Although the experiments reported here examine discrete priming and boosting steps which each required an administration of microencapsulated antigen, it will be possible to use the flexibility in controlled pulsatile release afforded by the microcapsule delivery system to design a single time of administration regimen which will stimulate maximum concurrent systemic and secretory immunity. As an example, microencapsulated antigen could be administered by both injection and ingestion during a single visit to a physician. By varying the lactide to glycolide ratio in the two doses, the systemically administered dose could be released within a few days to prime the immune system, and the second (oral) dose could be released in the Peyer's patches at a later time to stimulate a boosted mucosal response.

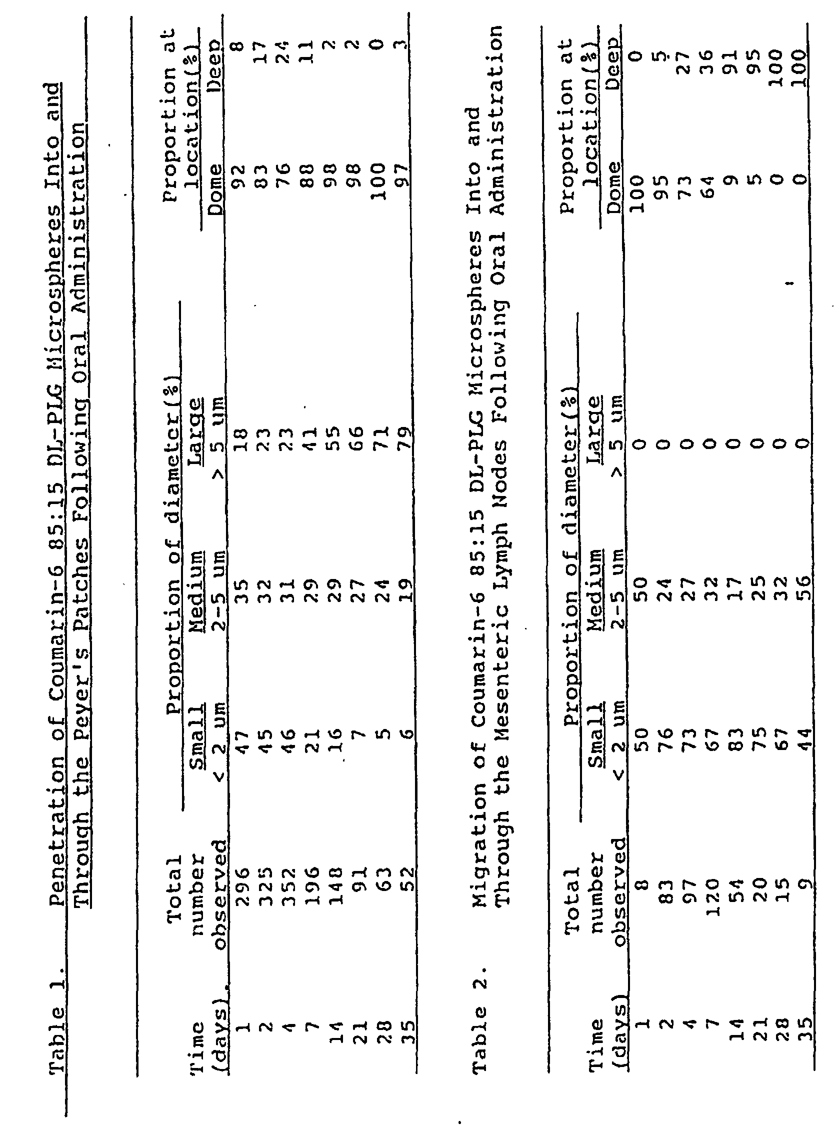

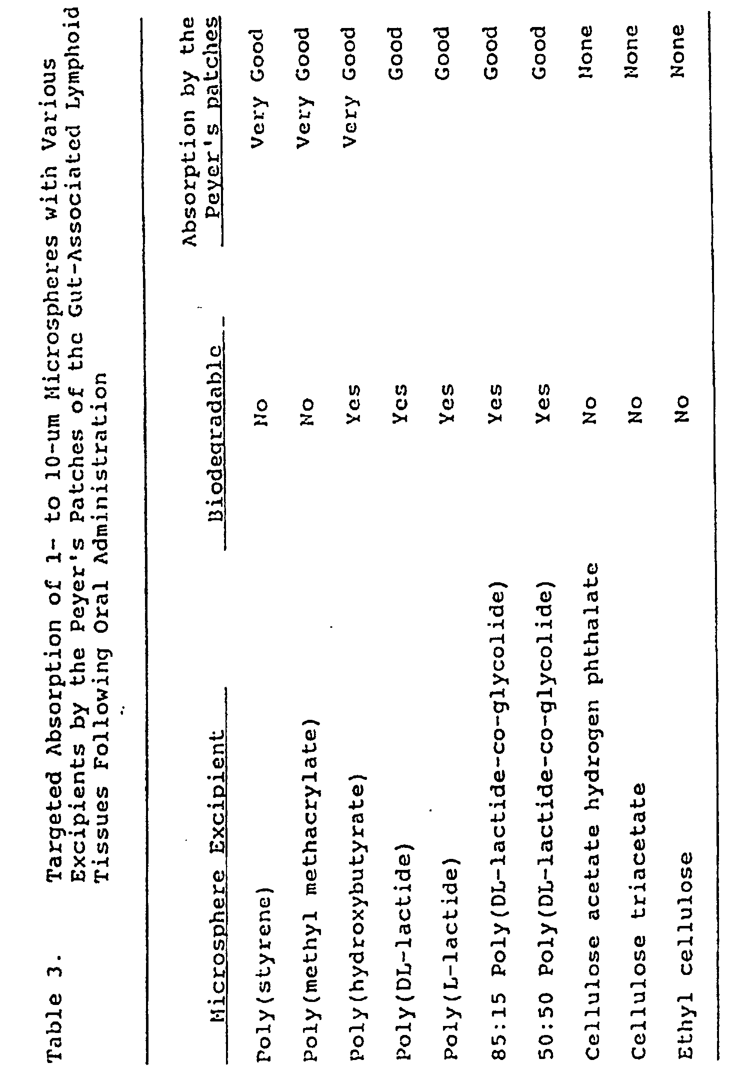

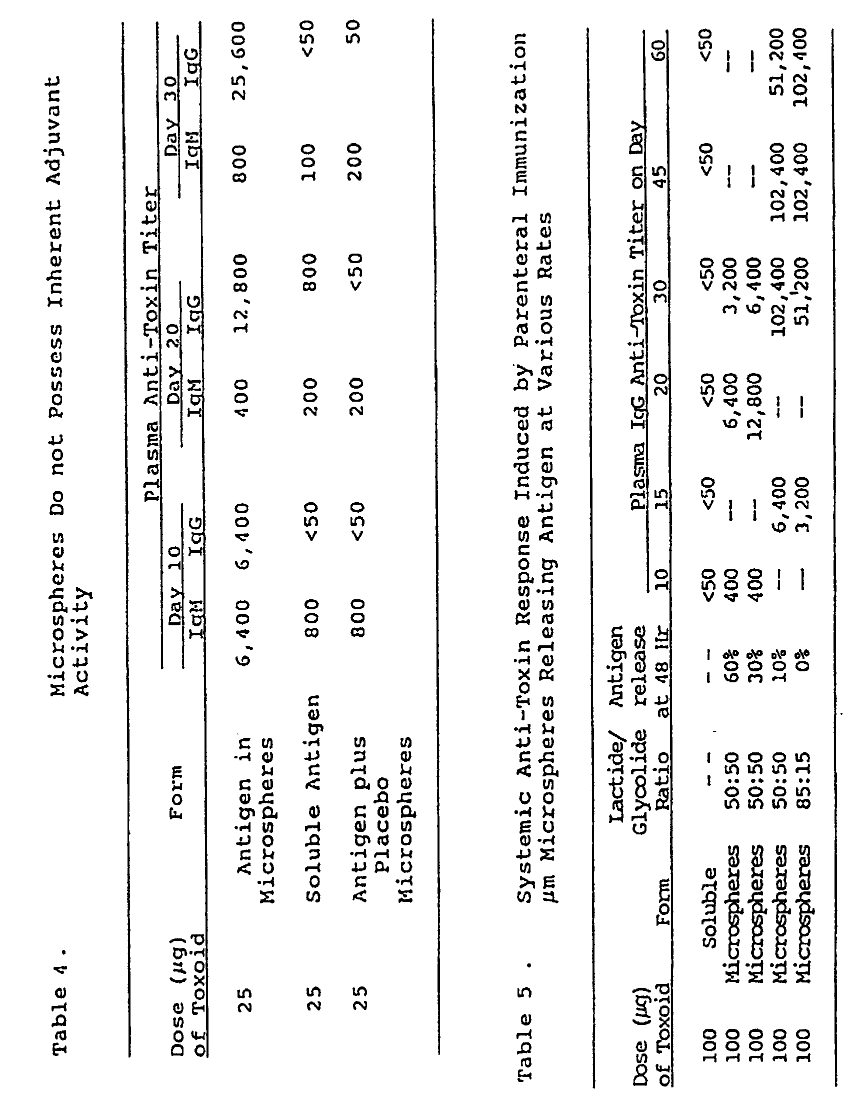

**Table 6.**

| Results of CPE Inhibition Assays on Serum Samples from the JE Vaccine Immunization Studies | | | |
|---|---|---|---|
| | Dilution of serum capable of reducing virus-induced CPE by 50% on Day | | |
| Animal | 21 | 49 | 77 |
| Group 1 = Untreated Controls | | | |
| GMI^{a} | <10 | 11 | 11 |
| Average | <10 | 11 | 11 |
| Maximum | <10 | 16 | <20 |
| Minimum | <10 | <10 | <10 |

| Group 2 = 3.0 mg unencapsulated JE vaccine IP on Day 10 | | | |
|---|---|---|---|
| GMT | 44 | 73 | 50 |
| Average | 55 | 95 | 71 |
| Maximum | 127 | 254 | 160 |
| Minimum | <10 | 13 | <10 |

| Group 3 = 3.0 mg unencapsulated JE vaccine IP on Days 0, 14 and 42 | | | |
|---|---|---|---|
| GMT | 507 | 3,880 | 1,576 |
| Average | 934 | 5,363 | 2,951 |
| Maximum | 4,064 | >10,240 | >10,240 |
| Minimum | 160 | 806 | 254 |

| Group 4 = 3.0 mg unencapsulated + 3.0 mg microencapsulated JE vaccine IP on Dav 0 | | | |
|---|---|---|---|
| GMT | 77 | 718 | 1,341 |
| Average | 803 | 1,230 | 2,468 |
| Maximum | 320 | 5,120 | 10,240 |
| Minimum | 13 | 160 | 254 |

| | | | |
|---|---|---|---|
| ^{a}GMT = Geometric mean titers. | | | |

**Table 7.**

| Results of Plaque-Reduction Assays on Pooled Serum Samples from the JE Vaccine Immunization Studies | | | | |
|---|---|---|---|---|
| | | | Serum dilution to reach | |
| Group | Treatment | Day | 50% endpoint | 80% endpoint |
| 1^{a} | Controls | 0 | <10 | <10 |
| 1 | Controls | 14 | <10 | <10 |
| 1 | Controls | 21 | <10 | <10 |
| 1 | Controls | 42 | <10 | <10 |
| 1 | Controls | 49 | <10 | <10 |
| 1 | Controls | 84 | <10 | <10 |
| | | | | |
| 2_{b} | Unencapsulated JE | 0 | <10 | <10 |
| 2 | Unencapsulated JE | 14 | 160 | 20 |
| 2 | Unencapsulated JE | 21 | ND^{c} | ND |
| 2 | Unencapsulated JE | 42 | 320 | 80 |
| 2 | Unencapsulated JE | 49 | 320 | 40 |
| 2 | Unencapsulated JE | 84 | 640 | 160 |
| | | | | |
| 3^{d} | Unencapsulated JE | 0 | <10 | <10 |
| 3 | Unencapsulated JE | 14 | 160 | 40 |
| 3 | Unencapsulated JE | 21 | 2,560 | 640 |
| 3 | Unencapsulated JE | 42 | 1,280 | 640 |
| 3 | Unencapsulated JE | 49 | 5,120 | 2,560 |
| 3 | Unencapsulated JE | 84 | 2,560 | 1,280 |
| | | | | |
| 4^{e} | Microencapsulated JE | 0 | <10 | <10 |
| 4 | Microencapsulated JE | 14 | 160 | 20 |
| 4 | Microencapsulated JE | 21 | 320 | 80 |
| 4 | Microencapsulated JE | 42 | 5,120 | 640 |
| 4 | Microencapsulated JE | 49 | 5,120 | 640 |
| 4 | Microencapsulated JE | 84 | 10,000 | 2,560 |

| | | | | |
|---|---|---|---|---|
| ^{a}Untreated controls. | | | | |
| ^{b}Animals received 3.0 mg of unencapsulated JE vaocine IP on Day 0. | | | | |
| ^{c}ND = Not determined (insufficient sample quantity). | | | | |
| ^{d}Animals received 3.0 mg of unencapsulated JE vaccine IP on Day 0, 14 and 42. | | | | |
| ^{e}Animals received 3.0 mg of unencapsulated and 3.0 mg of microencapsulated JE vaccine IP on Day 0. | | | | |

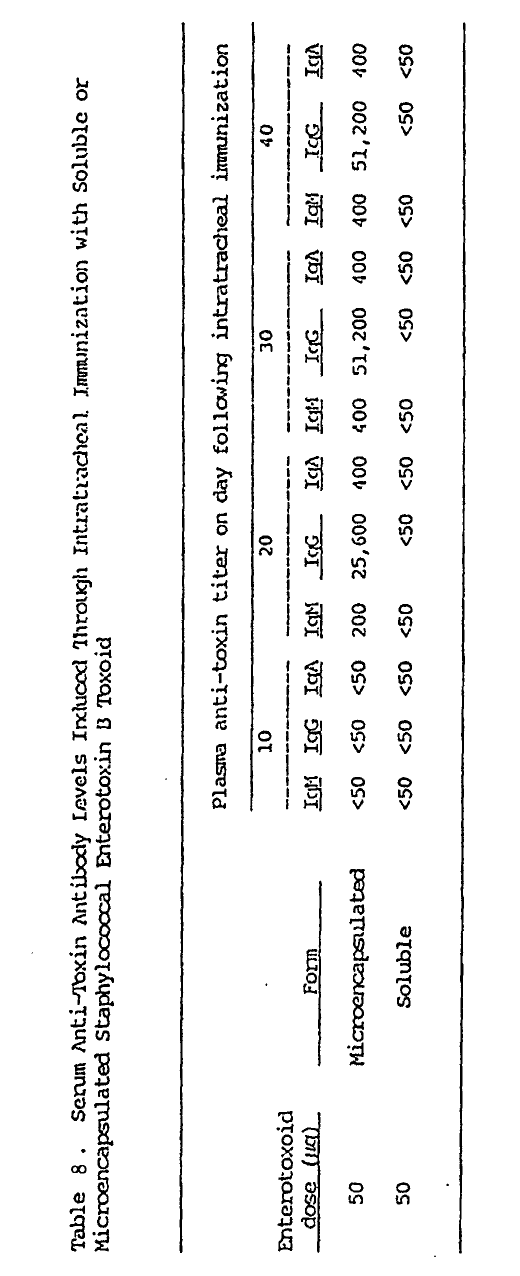

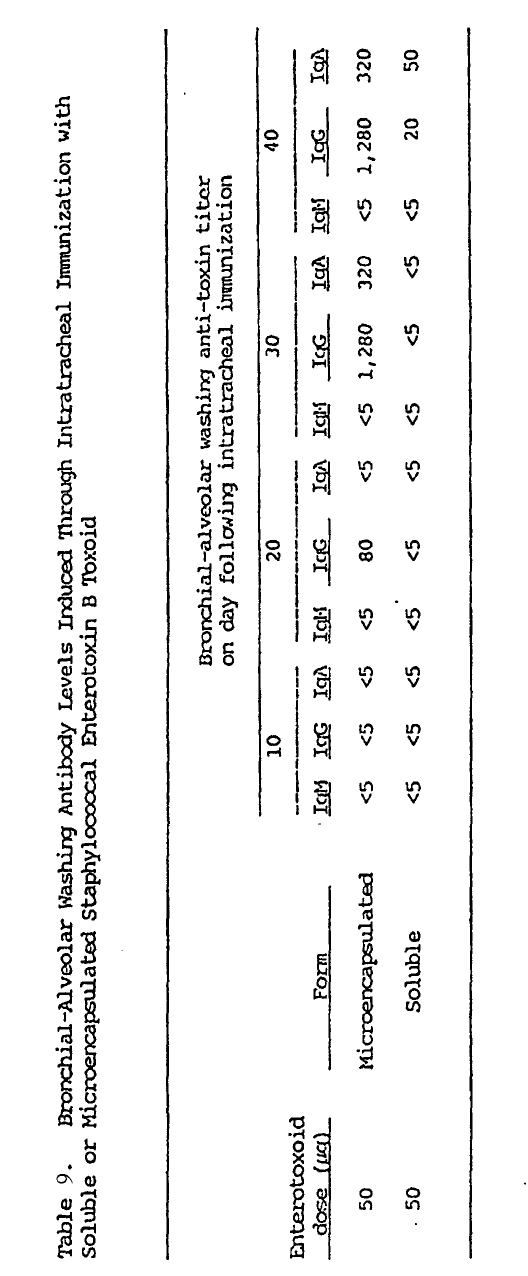

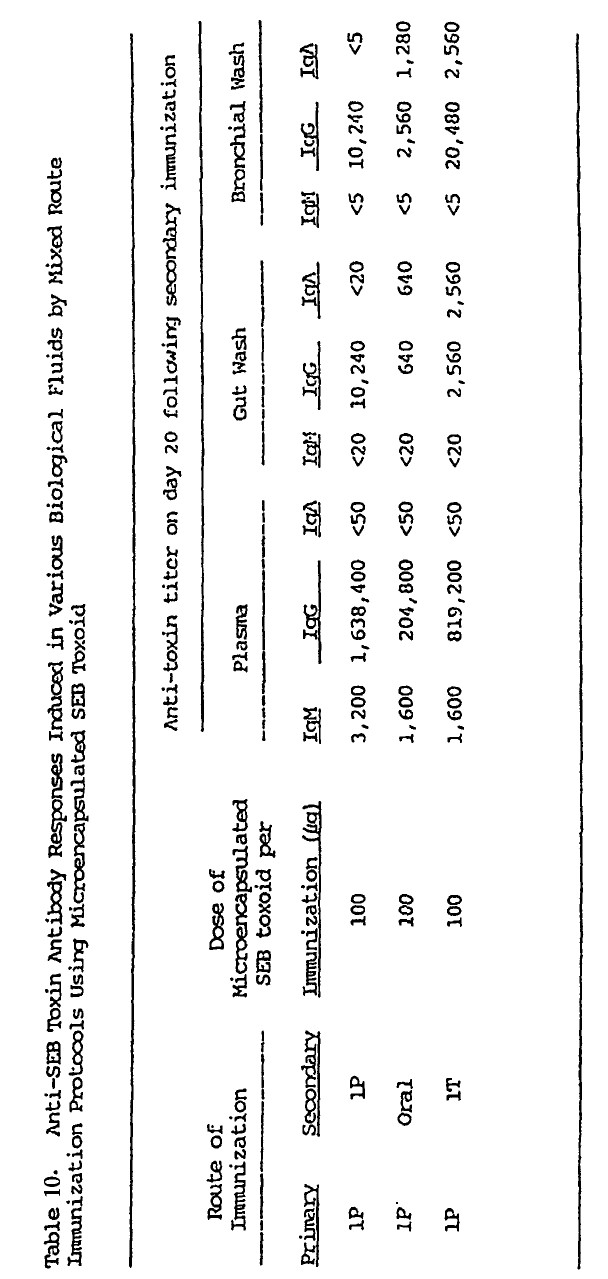

| Comparative Table 11 Toxin-Specific IgA Antibodies in the Saliva and Gut Fluids of Mice on Days 10 and 20 After Tertiary Oral Immunization with Soluble or Microencapsulated Enterotoxoid | | | | | |
|---|---|---|---|---|---|
| | | IgA anti-enterotoxoin titer following tertiary oral immunization | | | |
| Enterotoxoid dose (µg) per immunization | | Day 10 | | Day 20 | |
| | Form | Saliva | Gut Wash | Saliva | Gut Wash |
| 100 | Microspheres | 1,280 | 1,024 | 640 | 256 |
| 100 | Microspheres Soluble | 40 | <8 | 10 | <8 |

## Claims (Claims for the following Contracting State(s): AT, BE, CH, LI, DE, FR, GB, IT, LU, NL, SE)

1. Use of biocompatible microcapsules comprising an immunogenic agent encapsulated in a biocompatible excipient and having a size of less than approximately 10 µm for preparing a nasal, rectal, ophthalmic, intratracheal or inhalation preparation for delivering the immunogenic agent to mucosally associated lymphoreticular tissue of a human or other animal.

2. The use of claim 1 wherein a nasal, intratracheal or inhalation preparation is used.

3. The use of claim 1 or claim 2 wherein the microcapsules have a size between approximately 1 µm and approximately 10 µm.

4. Use according to one of claims 1 to 3 for delivery of immunogenic agent to folliculi lymphati aggregati in the respiratory tract, genitourinary tract and other mucosal tissues of the body other than in the gastrointestinal tract.

5. Use according to one of claims 1 to 4 for absorption and passage through the mucosally associated lymphoreticular tissue, wherein the microcapsules have a size from 1 µm to less than 5 µm.

6. Use according to one of claims 1 to 4 for absorption and retention by the mucosally associated lymphoreticular tissue, wherein the microcapsules have a size from 5 µm to less than 10 µm.

7. Use according to one of claims 1 to 6 for stimulating the immune system of a human or other animal.

8. Use according to one of claims 1 to 7 wherein the immunogenic agent is an antigen, an allergen, a lymphokine, a cytokine, a monokine or an immunomodulator.

9. Use according to claim 8 wherein the immunogenic agent is influenza antigen, Staphylococcus antigen, respiratory syncytial antigen, parainfluenza virus antigen, Hemophilos influenza antigen, Bordetella pertussis antigen, Neisseria gonorrhoeae antigen, Streptococcus pneumoniae antigen, Plasmodium falciparum antigen, helminthic pathogen antigen, viral antigen, bacterial antigen, fungal antigen or protozoan antigen or an antigen to vaccinate against allergy.

10. Use according to claim 9 wherein the immunogenic agent comprises an influenza virus or staphylococcal enterotoxin B.

11. Use according to one of claims 1 to 10, wherein the excipient has a size and physical chemical properties such that the microcapsules are effectively reach and are selectively taken up by mucosally associated lymphoreticular tissue.

12. Use according of any of claims 1 to 11, wherein the excipient comprises a polymer or copolymer.

13. Use according of any of claims 1 to 12, wherein the excipient comprises a biodegradable polymer or copolymer.

14. Use according to any of claims 1 to 13, wherein the excipient is a hydrophobic polymer.

15. Use according to claim 10 to 14, wherein the excipient comprises a poly(DL-lactide-co-glycolide), a poly(L-lactide), a poly(DL-lactide), a poly(glycolide), a copolyoxalate, a polycaprolactone, a poly(lactide-co-caprolactone), a poly(esteramide), a polyorthoester, a poly(β-hydroxybutyric acid) or a polyanhydride or a mixture thereof.

16. A composition for stimulating the immune response of a human or other animal, comprising a mixture of a first amount of biocompatible microcapsules having a size of less than approximately 10 µm and containing an immunogenic agent encapsulated in a first biocompatible excipient and a second amount of biocompatible microcapsules having a size of greater than 10 µm and containing an immunogenic agent encapsulated in a second biocompatible excipient, the first microcapsules providing a primary immunological response and the second microcapsules releasing the agent contained in the second microcapsules in a pulsed manner to potentiate a subsequent immunological response.

17. A composition according to claim 16, wherein the first microcapsules have a size between 1 µm and 10 µm.

18. A composition according to one of claims 16-17, which comprises third and optionally further biocompatible microcapsules containing an immunogenic agent, the third and further microcapsules releasing the agent contained therein in a pulsed manner after the second microcapsules release the agent contained therein.

19. A composition according to one of claims 16-18, wherein two different biodegradable polymers are used as first and second excipients and wherein the biodegradation rate of the first polymeric excipient varies from the biodegradation rate of the second polymeric excipient.

20. Use of a composition comprising a mixture of a first amount of biocompatible microcapsules having a size of less than 10 µm and containing an immunogenic agent encapsulated in a biocompatible excipient and a second amount of biocompatible microcapsules having a size of greater than 10 µm and containing an immunogenic agent encapsulated in a biocompatible excipient, the first microcapsules providing a primary immunological response and the second microcapsules releasing the agent contained in the second microcapsules in a pulsed manner, for preparing a preparation for inducing an immunological response in a human or other animal.

21. Use according to claim 10, wherein the first and second and optionally further microcapsules are administered in one preparation.

22. Use for the preparation of a combined composition comprising first biocompatible microcapsules comprising an immunogenic agent encapsulated in a biocompatible excipient and having a size of between 1µm and 10µm for administration to a human or other animal by a first route and second biocompatible microcapsules comprising the immunogenic agent encapsulated in a biocompatible excipient and having a size of between 1 µm and 10 µm for administration to a human or other animal by a second route different from the first route for potentiating the immune response of a human or other animal.

## Claims (Claims for the following Contracting State(s): ES, GR)

1. Use of biocompatible microcapsules comprising an immunogenic agent encapsulated in a biocompatible excipient and having a size of less than approximately 10 µm for preparing a nasal, rectal, ophthalmic, intratracheal or inhalation preparation for delivering the immunogenic agent to mucosally associated lymphoreticular tissue of a human or other animal.

2. The use of claim 1 wherein a nasal, intratracheal or inhalation preparation is used.

3. The use of claim 1 or claim 2 wherein the have a size between approximately 1 µm and approximately 10 µm.

4. Use according to one of claims 1 to 3 for delivery of immunogenic agent to folliculi lymphati aggregati in the respiratory tract, genitourinary tract and other mucosal tissues of the body other than in the gastrointestinal tract.

5. Use according to one of claims 1 to 4 for absorption and passage through the mucosally associated lymphoreticular tissue, wherein the microcapsules have a size from 1 µm to less than 5 µm.

6. Use according to one of claims 1 to 4 for absorption and retention by the mucosally associated lymphoreticular tissue, wherein the microcapsules have a size from 5 µm to less than 10 µm.

7. Use according to one of claims 1 to 6 for stimulating the immune system of a human or other animal.

8. Use according to one of claims 1 to 7 wherein the immunogenic agent is an antigen, an allergen, a lymphokine, a cytokine, a monokine or an immunomodulator.

9. Use according to claim 8 wherein the immunogenic agent is influenza antigen, Staphylococcus antigen, respiratory syncytial antigen, parainfluenza virus antigen, Hemophilos influenza antigen, Bordetella pertussis antigen, Neisseria gonorrhoeae antigen, Streptococcus pneumoniae antigen, Plasmodium falciparum antigen, helminthic pathogen antigen, viral antigen, bacterial antigen, fungal antigen or protozoan antigen or an antigen to vaccinate against allergy.

10. Use according to claim 9 wherein the immunogenic agent comprises an influenza virus or staphylococcal enterotoxin B.

11. Use according to one of claims 1 to 10, wherein the excipient has a size and physical chemical properties such that the microcapsules are effectively reach and are selectively taken up by mucosally associated lymphoreticular tissue.

12. Use according of any of claims 1 to 11, wherein the excipient comprises a polymer or copolymer.

13. Use according of any of claims 1 to 12, wherein the excipient comprises a biodegradable polymer or copolymer.

14. Use according to any of claims 1 to 13, wherein the excipient is a hydrophobic polymer.

15. Use according to claim 10 to 14, wherein the excipient comprises a poly(DL-lactide-co-glycolide), a poly(L-lactide), a poly(DL-lactide), a poly(glycolide), a copolyoxalate, a polycaprolactone, a poly(lactide-co-caprolactone), a poly(esteramide), a polyorthoester, a poly(β-hydroxybutyric acid) or a polyanhydride or a mixture thereof.

16. A method of making a composition for stimulating the immune response of a human or other animal, comprising preparing a mixture of a first amount of biocompatible microcapsules having a size of less than approximately 10 µm and containing an immunogenic agent encapsulated in a first biocompatible excipient and a second amount of biocompatible microcapsules having a size of greater than 10 µm and containing an immunogenic agent encapsulated in a second biocompatible excipient, the first microcapsules providing a primary immunological response and the second microcapsules releasing the agent contained in the second microcapsules in a pulsed manner to potentiate a subsequent immunological response.

17. The method according to claim 16, wherein the first microcapsules have a size between 1 µm and 10 µm.

18. The method according to one of claims 16 or 17, wherein the mixture comprises third and optionally further biocompatible microcapsules containing an immunogenic agent, the third and further microcapsules releasing the agent contained therein in a pulsed manner after the second microcapsules release the agent contained therein.

19. The method according to one of claims 16 to 18, wherein two different biodegradable polymers are used as first and second excipients and wherein the biodegradation rate of the first excipient varies from the biodegradation rate of the polymer of the second excipient.

20. Use of a composition comprising a mixture of a first amount of biocompatible microcapsules having a size of less than 10 µm and containing an immunogenic agent encapsulated in a biocompatible excipient and a second amount of biocompatible microcapsules having a size of greater than 10 µm and containing an immunogenic agent encapsulated in a biocompatible excipient, the first microcapsules providing a primary immunological response and the second microcapsules releasing the agent contained in the second microcapsules in a pulsed manner, for preparing a preparation for inducing an immunological response in a human or other animal.

21. Use according to claim 20, wherein the first and second and optionally further microcapsules are administered in one preparation.

22. Use for the preparation of a combined composition comprising first biocompatible microcapsules comprising an immunogenic agent encapsulated in a biocompatible excipient and having a size of between 1 µm and 10 µm for adminstration to a human or other animal by a first route and second biocomaptible microcapsules comprising the immunogenic agent encapsulated in a biocompatible excipient and having a size of between 1 µm and 10 µm for adminstration to a human or other animal by a second route different from the first route for potentiating the immune response of a human or other animal.

## Patentansprüche (Patentansprüche für folgende(n) Vertragsstaat(en): AT, BE, CH, LI, DE, FR, GB, IT, LU, NL, SE)

1. Verwendung von biokompatiblen Mikrokapseln mit einem immunogenen Mittel, das in einen biokompatiblen Hilfsstoff eingekapselt ist und mit einer Größe von weniger als ungefähr 10 µm zur Herstellung eines nasalen, rektalen, ophtalmischen, intratrachealen oder Inhalationspräparats zur Abgabe des immunogenen Mittels an Mukosa-assoziiertes lymphoides Gewebe eines Menschen oder anderen Tiers.

2. Verwendung nach Anspruch 1, wobei ein nasales, intratracheales oder Inhalationspräparat verwendet wird.

3. Verwendung nach Anspruch 1 oder Anspruch 2, wobei die Mikrokapseln eine Größe zwischen ungefähr 1 µm und ungefähr 10 µm haben.

4. Verwendung nach einem der Ansprüche 1 bis 3 zur Abgabe eines immunogenen Mittels an die Folliculi lymphati aggregati in den Atemwegen, dem Urogenitaltrakt und anderen Schleimhautgeweben des Körpers außer dem Gastrointestinaltrakt.

5. Verwendung nach einem der Ansprüche 1 bis 4 zur Absorption an und Passage durch das Mukosa-assoziierte lymphoide Gewebe, wobei die Mikrokapseln eine Größe von 1 µm bis weniger als 5 µm haben.

6. Verwendung nach einem der Ansprüche 1 bis 4 zur Absorption und Retention durch das Mukosa-assoziierte lymphoide Gewebe, wobei die Mikrokapseln eine Größe von 5 µm bis weniger als 10 µm haben.

7. Verwendung nach einem der Ansprüche 1 bis 6 zur Stimulierung des Immunsystems eines Menschen oder anderen tierischen Wesens.

8. Verwendung nach einem der Ansprüche 1 bis 7, wobei das immunogene Mittel ein Antigen, Allergen, Lymphokin, Cytokin, Monokin oder Immunmodulator ist.

9. Verwendung nach Anspruch 8, wobei das immunogene Mittel Influenza-Antigen, Staphylococcus-Antigen, Respiratory-Syncytial-Antigen, Parainfluenza-Virus-Antigen, Hämophilos influenza-Antigen, Bordetella pertussis-Antigen, Neisseria gonorrhoeae-Antigen, Streptococcus pneumoniae-Antigen, Plasmodium falciparum-Antigen, Helminth-Pathogen-Antigen, Virus-Antigen, Bakterien-Antigen, Pilz-Antigen oder Protozoen-Antigen oder ein Antigen, um gegen Allergie zu impfen, ist.

10. Verwendung nach Anspruch 9, wobei das immunogene Mittel ein Influenzavirus oder Staphylococcus Enterotoxin B enthält.

11. Verwendung nach einem der Ansprüche 1 bis 10, wobei der Hilfsstoff eine solche Größe und solche physikalisch-chemischen Eigenschaften hat, dass die Mikrokapseln wirksam das Mukosa-assoziierte lymphoide Gewebe erreichen und von diesem selektiv aufgenommen werden.

12. Verwendung nach einem der Ansprüche 1 bis 11, wobei der Hilfsstoff ein Polymer oder Copolymer aufweist.

13. Verwendung nach einem der Ansprüche 1 bis 12, wobei der Hilfsstoff ein biologisch abbaubares Polymer oder Copolymer aufweist.

14. Verwendung nach einem der Ansprüche 1 bis 13, wobei der Hilfsstoff ein hydrophobes Polymer ist.

15. Verwendung nach Anspruch 10 bis 14, wobei der Hilfsstoff ein Poly(DLlactid-co-glycolid), Poly(L-lactid), Poly(DL-lactid), Poly(glycolid), Copolyoxalat, Polycaprolacton, Poly(iactid-co-caprolacton), Poly(esteramid), Polyorthoester, Poly(β-hydroxybuttersäure) oder Polyanhydrid oder eine Mischung davon enthält.

16. Zusammensetzung zur Stimulierung der Immunantwort eines Menschen oder anderen tierischen Wesens enthaltend eine Mischung aus einer ersten Menge von biokompatiblen Mikrokapseln mit einer Größe von weniger als ungefähr 10 µm, die ein immunogenes Mittel enthalten, eingekapselt in einen ersten biokompatiblen Hilfsstoff, und einer zweiten Menge biokompatibler Mikrokapseln mit einer Größe von mehr als 10 µm, die ein immunogenes Mittel eingekapselt in einen zweiten biokompatiblen Hilfsstoff enthalten, wobei die ersten Mikrokapseln eine erste immunologische Antwort liefern und die zweiten Mikrokapseln das Mittel, das in den zweiten Mikrokapseln enthalten ist, in gepulster Art und Weise freisetzen, um eine nachfolgende immunologische Antwort zu potenzieren.

17. Zusammensetzung nach Anspruch 16, wobei die ersten Mikrokapseln eine Größe zwischen 1 µm und 10 µm haben.

18. Zusammensetzung nach einem der Ansprüche 16 bis 17, die dritte und ggf. weitere biokompatible Mikrokapseln enthält, die ein immunogenes Mittel enthalten, wobei die dritten und weiteren Mikrokapseln das Mittel, das darin enthalten ist, in gepulster Art und Weise freisetzen, nachdem die zweiten Mikrokapseln das darin enthaltene Mittel freigesetzt haben.

19. Zusammensetzung nach einem der Ansprüche 16 bis 18, wobei zwei verschiedene biologisch abbaubare Polymere als erste und zweite Hilfsstoffe verwendet werden und wobei die biologische Abbaurate des ersten polymeren Hilfsstoffs gegenüber der biologischen Abbaurate des zweiten polymeren Hilfsstoffs variiert.

20. Verwendung einer Zusammensetzung mit einer Mischung aus einer ersten Menge biokompatibler Mikrokapseln mit einer Größe von weniger als 10 µm, die eingekapselt in einen biokompatiblen Hilfsstoff ein immunogenes Mittel enthalten, und einer zweiten Menge biokompatibler Mikrokapseln mit einer Größe von mehr als 10 µm, die eingekapselt in einen biokompatiblen Hilfsstoff ein immunogenes Mittel enthalten, wobei die ersten Mikrokapseln eine primäre immunologische Antwort liefern und die zweiten Mikrokapseln das Mittel, das in den zweiten Mikrokapseln enthalten ist, in gepulster Art und Weise freisetzen, zur Herstellung eines Präparats, um eine immunologische Antwort bei einem Menschen oder anderen tierischen Wesens zu induzieren.

21. Verwendung nach Anspruch 20, wobei die ersten und zweiten und ggf. weitere Mikrokapseln in einem Präparat verabreicht werden.

22. Verwendung zur Herstellung einer kombinierten Zusammensetzung mit ersten biokompatiblen Mikrokapseln mit einem immunogenen Mittel eingekapselt in einem biokompatiblen Hilfsstoff und mit einer Größe zwischen 1 µm und 10 µm zur Verabreichung an einen Menschen oder ein anderes tierisches Wesen über einen ersten Weg und zweiten biokompatiblen Mikrokapseln mit dem immunogenen Mittel eingekapselt in einen biokompatiblen Hilfsstoff und mit einer Größe zwischen 1 µm und 10 µm zur Verabreichung an einen Menschen oder ein anderes tierisches Wesen auf einem zweiten Weg, der vom ersten Weg verschieden ist, um die Immunantwort eines Menschen oder anderen tierischen Wesens zu verstärken bzw. zu potenzieren.

## Patentansprüche (Patentansprüche für folgende(n) Vertragsstaat(en): ES, GR)

1. Verwendung von biokompatiblen Mikrokapseln mit einem immunogenen Mittel, das in einen biokompatiblen Hilfsstoff eingekapselt ist und mit einer Größe von weniger als ungefähr 10 µm zur Herstellung eines nasalen, rektalen, ophtalmischen, intratrachealen oder Inhalationspräparats zur Abgabe des immunogenen Mittels an Mukosa-assoziiertes lymphoides Gewebe eines Menschen oder anderen tierischen Wesens.

2. Verwendung nach Anspruch 1, wobei ein nasales, intratracheales oder Inhalationspräparat verwendet wird.

3. Verwendung nach Anspruch 1 oder Anspruch 2, wobei die Mikrokapseln eine Größe zwischen ungefähr 1 µm und ungefähr 10 µm haben.

4. Verwendung nach einem der Ansprüche 1 bis 3 zur Abgabe eines immunogenen Mittels an Folliculi lymphati aggregati in den Atemwegen, dem Urogenitaltrakt und anderen Schleimhautgeweben des Körpers außer dem Gastrointestinaltrakt.

5. Verwendung nach einem der Ansprüche 1 bis 4 zur Absorption an und Passage durch das Mukosa-assoziierte lymphoide Gewebe, wobei die Mikrokapseln eine Größe von 1 µm bis weniger als 5 µm haben.

6. Verwendung nach einem der Ansprüche 1 bis 4 zur Absorption und Retention durch das Mukosa-assoziierte lymphoide Gewebe, wobei die Mikrokapseln eine Größe von 5 µm bis weniger als 10 µm haben.

7. Verwendung nach einem der Ansprüche 1 bis 6 zur Stimulierung des Immunsystems eines Menschen oder anderen tierischen Wesens.

8. Verwendung nach einem der Ansprüche 1 bis 7, wobei das immunogene Mittel ein Antigen, Allergen, Lymphokin, Cytokin, Monokin oder Immunmodulator ist.

9. Verwendung nach Anspruch 8, wobei das immunogene Mittel Influenza-Antigen, Staphylococcus-Antigen, Respiratory-Syncytial-Antigen, Paralnfluenza-Virus-Antigen, Hämophilos influenza-Antigen, Bordetella pertussis-Antigen, Neisseria gonorrhoeae-Antigen, Streptococcus pneumoniae-Antigen, Plasmodium falciparum-Antigen, Helminth-Pathogen-Antigen, Virus-Antigen, Bakterien-Antigen, Pilz-Antigen oder Protozoen-Antigen oder ein Antigen, um gegen Allergie zu impfen, ist.

10. Verwendung nach Anspruch 9, wobei das immunogene Mittel ein Influenzavirus oder Staphylococcus Enterotoxin B enthält.

11. Verwendung nach einem der Ansprüche 1 bis 10, wobei der Hilfsstoff eine solche Größe und solche physikalisch-chemischen Eigenschaften hat, dass die Mikrokapseln wirksam das Mukosa-assoziierte lymphoide Gewebe erreichen und von diesem selektiv aufgenommen werden.

12. Verwendung nach einem der Ansprüche 1 bis 11, wobei der Hilfsstoff ein Polymer oder Copolymer aufweist.

13. Verwendung nach einem der Ansprüche 1 bis 12, wobei der Hilfsstoff ein biologisch abbaubares Polymer oder Copolymer aufweist.

14. Verwendung nach einem der Ansprüche 1 bis 13, wobei der Hilfsstoff ein hydrophobes Polymer ist.

15. Verwendung nach Anspruch 10 bis 14, wobei der Hilfsstoff ein Poly(DLlactid-co-glycolid), Poly(L-lactid), Poly(DL-lactid), Poly(glycolid), Copolyoxalat, Polycaprolacton, Poly(lactid-co-caprolacton), Poly(esteramid), Polyorthoester, Poly(β-hydroxybuttersäure) oder Polyanhydrid oder eine Mischung davon enthält.

16. Verfahren zur Herstellung einer Zusammensetzung zur Stimulierung der Immunantwort eines Menschen oder anderen tierischen Wesens, das umfasst, dass eine Mischung aus einer ersten Menge von biokompatiblen Mikrokapseln mit einer Größe von weniger als ungefähr 10 µm, die ein immunogenes Mittel eingekapselt in einen ersten biokompatiblen Hilfsstoff enthalten, und einer zweiten Menge biokompatibler Mikrokapseln mit einer Größe von mehr als 10 µm, die ein immunogenes Mittel eingekapselt in einen zweiten biokompatiblen Hilfsstoff enthalten, hergestellt wird, wobei die ersten Mikrokapseln eine erste immunologische Antwort liefern und die zweiten Mikrokapseln das Mittel, das in den zweiten Mikrokapseln enthalten ist, in gepulster Art und Weise freisetzen, um eine nachfolgende immunologische Antwort zu potenzieren.

17. Verfahren nach Anspruch 16, wobei die ersten Mikrokapseln eine Größe zwischen 1 µm und 10 µm haben.

18. Verfahren nach einem der Ansprüche 16 bis 17, wobei die Mischung dritte und ggf. weitere biokompatible Mikrokapseln enthält, die ein immunogenes Mittel enthalten, wobei die dritten und weiteren Mikrokapseln das Mittel, das darin enthalten ist, in gepulster Art und Weise freisetzen, nachdem die zweiten Mikrokapseln das darin enthaltene Mittel freigesetzt haben.

19. Verfahren nach einem der Ansprüche 16 bis 18, wobei zwei verschiedene biologisch abbaubare Polymere als erste und zweite Hilfsstoffe verwendet werden und wobei die biologische Abbaurate des ersten Hilfsstoffs gegenüber der biologischen Abbaurate des Polymers des zweiten Hilfsstoffs variiert.

20. Verwendung einer Zusammensetzung mit einer Mischung aus einer ersten Menge biokompatibler Mikrokapseln mit einer Größe von weniger als 10 µm, die eingekapselt in einen biokompatiblen Hiffsstoff ein immunogenes Mittel enthalten, und einer zweiten Menge biokompatibler Mikrokapseln mit einer Größe von mehr als 10 µm, die eingekapselt in einen biokompatiblen Hilfsstoff ein immunogenes Mittel enthalten, wobei die ersten Mikrokapseln eine primäre immunologische Antwort liefern und die zweiten Mikrokapseln das Mittel, das in den zweiten Mikrokapseln enthalten ist, in gepulster Art und Weise freisetzen, zur Herstellung eines Präparats, um eine immunologische Antwort bei einem Menschen oder anderen tierischen Wesen zu induzieren.

21. Verwendung nach Anspruch 20, wobei die ersten und zweiten und ggf. weitere Mikrokapseln in einem Präparat verabreicht werden.

22. Verwendung zur Herstellung einer kombinierten Zusammensetzung mit ersten biokompatiblen Mikrokapseln mit einem immunogenen Mittel eingekapselt in einem biokompatiblen Hilfsstoff und mit einer Größe zwischen 1 µm und 10 µm zur Verabreichung an einen Menschen oder ein anderes tierisches Wesen über einen ersten Weg und zweiten biokompatiblen Mikrokapseln mit dem immunogenen Mittel eingekapselt in einen biokompatiblen Hilfsstoff und mit einer Größe zwischen 1 µm und 10 µm zur Verabreichung an einen Menschen oder ein anderes tierisches Wesen auf einem zweiten Weg, der vom ersten Weg verschieden ist, um die Immunantwort eines Menschen oder anderen tierischen Wesens zu verstärken bzw. zu potenzieren.

## Revendications (Revendications pour l'(les) Etat(s) contractant(s) suivant(s): AT, BE, CH, LI, DE, FR, GB, IT, LU, NL, SE)

1. Utilisation de micro-capsules biocompatibles comprenant un agent immunogène encapsulé dans un excipient biocompatible et présentant une taille inférieure à approximativement 10 µm pour préparer une préparation nasale, rectale, ophtalmique, intratrachéale ou d'inhalation pour délivrer l'agent immunogène à un tissu lymphoréticulaire associé aux muqueuses d'un être humain ou autre animal.

2. Utilisation selon la revendication 1, dans laquelle une préparation nasale, intratrachéale ou d'inhalation est utilisée.

3. Utilisation selon la revendication 1 ou la revendication 2, dans laquelle les micro-capsules présentent une taille entre approximativement 1 µm et approximativement 10 µm.

4. Utilisation selon l'une des revendications 1 à 3, pour une délivrance d'agent immunogène à des agrégats de follicules lymphatiques dans les voies respiratoires, les voies génito-urinaires et autres tissus muqueux du corps autres que dans les voies gastro-intestinales.

5. Utilisation selon l'une des revendications 1 à 4, pour une absorption et un passage par le tissu lymphoréticulaire associé aux muqueuses, dans laquelle les micro-capsules présentent une taille de 1 µm à moins de 5 µm.

6. Utilisation selon l'une des revendications 1 à 4, pour une absorption et une rétention par le tissu lymphoréticulaire associé aux muqueuses, dans laquelle les micro-capsules présentent une taille de 5 µm à moins de 10 µm.

7. Utilisation selon l'une des revendications 1 à 6, destinée à stimuler le système immunitaire d'un être humain ou autre animal.

8. Utilisation selon l'une des revendications 1 à 7, dans laquelle l'agent immunogène est un antigène, un allergène, une lymphokine, une cytokine, une monokine ou un immunorégulateur.

9. Utilisation selon la revendication 8, dans laquelle l'agent immunogène est un antigène de la grippe, un antigène de Staphylocoque, un antigène syncytial respiratoire, un antigène de virus para-influenza, un antigène d'Hémophilus influenzae, un antigène de Bordetella pertussis, un antigène de Neisseria gonorrhoeae, un antigène de Streptococcus pneumoniae, un antigène de Plasmodium falciparum, un antigène d'agent pathogène helminthique, un antigène viral, un antigène bactérien, un antigène fongique ou un antigène de protozoose ou un antigène pour une vaccination contre une allergie.

10. Utilisation selon la revendication 9, dans laquelle l'agent immunogène comprend un virus de la grippe ou une entérotoxine staphylococcique B.

11. Utilisation selon l'une des revendications 1 à 10, dans laquelle l'excipient présente une taille et des propriétés chimico-physiques telles que les micro-capsules atteignent effectivement et sont prélevées sélectivement par un tissu lymphoréticulaire associé aux muqueuses.

12. Utilisation selon l'une quelconque des revendications 1 à 11, dans laquelle l'excipient comprend un polymère ou un copolymère.

13. Utilisation selon l'une quelconque des revendications 1 à 12, dans laquelle l'excipient comprend un polymère ou un copolymère biodégradable.

14. Utilisation selon l'une quelconque des revendications 1 à 13, dans laquelle l'excipient est un polymère hydrophobe.

15. Utilisation selon les revendications 10 à 14, dans laquelle l'excipient comprend un poly(DL-lactide-co-glycolide), un poly(L-lactide), un poly(DL-lactide), un poly(glycolide), un copolyoxalate, une polycaprolactone, une poly(lactide-co-caprolactone), un poly(estéramide), un polyorthoester, un acide poly(β-hydroxybutyrique) ou un polyanhydride ou un mélange de ceux-ci.

16. Composition destinée à stimuler la réponse immunitaire d'un être humain ou autre animal, comprenant un mélange d'une première quantité de micro-capsules biocompatibles présentant une taille inférieure à approximativement 10 µm et contenant un agent immunogène encapsulé dans un premier excipient biocompatible et d'une seconde quantité de micro-capsules biocompatibles présentant une taille supérieure à 10 µ met contenant un agent immunogène encapsulé dans un second excipient biocompatible, les premières micro-capsules fournissant une réponse immunologique principale et les secondes micro-capsules libérant l'agent contenu dans les secondes micro-capsules de manière pulsée afin de potentialiser une réponse immunologique ultérieure.

17. Composition selon la revendication 16, dans laquelle les premières micro-capsules présentent une taille entre 1 µm et 10 µm.

18. Composition selon l'une des revendications 16 et 17, qui comprend des troisièmes et optionnellement d'autres micro-capsules biocompatibles contenant un agent immunogène, les troisièmes et autres micro-capsules libérant l'agent contenu dans celles-ci de manière pulsée après que les secondes micro-capsules libèrent l'agent contenu dans celles-ci.

19. Composition selon l'une des revendications 16 à 18, dans laquelle deux polymères biodégradables différents sont utilisés en tant que premier et second excipients et dans laquelle la vitesse de biodégradation du premier excipient polymère varie par rapport à la vitesse de biodégradation du second excipient polymère.

20. Utilisation d'une composition comprenant un mélange d'une première quantité de micro-capsules biocompatibles présentant une taille inférieure à 10 µm et contenant un agent immunogène encapsulé dans un excipient biocompatible et d'une seconde quantité de micro-capsules biocompatibles présentant une taille supérieure à 10 µm et contenant un agent immunogène encapsulé dans un excipient biocompatible, les premières micro-capsules fournissant une réponse immunologique principale et les secondes micro-capsules libérant l'agent contenu dans les secondes micro-capsules de manière pulsée, afin de préparer une préparation destinée à induire une réponse immunologique dans un être humain ou autre animal.

21. Utilisation selon la revendication 20, dans laquelle les première et seconde et optionnellement autres micro-capsules sont administrées dans une préparation.

22. Utilisation destinée à la préparation d'une composition combinée comprenant des premières micro-capsules biocompatibles comprenant un agent immunogène encapsulé dans un excipient biocompatible et présentant une taille entre 1 µm et 10 µm en vue d'une administration à un être humain ou autre animal par une première voie et des secondes micro-capsules biocompatibles comprenant l'agent immunogène encapsulé dans un excipient biocompatible et présentant une taille entre 1 µm et 10 µm en vue d'une administration à un être humain ou autre animal par une seconde voie différente de la première voie afin de potentialiser la réponse immunitaire d'un être humain ou autre animal.

## Revendications (Revendications pour l'(les) Etat(s) contractant(s) suivant(s): ES, GR)

1. Utilisation de micro-capsules biocompatibles comprenant un agent immunogène encapsulé dans un excipient biocompatible et présentant une taille inférieure à approximativement 10 µm pour préparer une préparation nasale, rectale, ophtalmique, intratrachéale ou d'inhalation pour délivrer l'agent immunogène à un tissu lymphoréticulaire associé aux muqueuses d'un être humain ou autre animal.

2. Utilisation selon la revendication 1, dans laquelle une préparation nasale, intratrachéale ou d'inhalation est utilisée.

3. Utilisation selon la revendication 1 ou la revendication 2, dans laquelle les micro-capsules présentent une taille entre approximativement 1 µm et approximativement 10 µm.

4. Utilisation selon l'une des revendications 1 à 3, pour une délivrance d'agent immunogène à des agrégats de follicules lymphatiques dans les voies respiratoires, les voies génito-urinaires et autres tissus muqueux du corps autres que dans les voies gastro-intestinales.

5. Utilisation selon l'une des revendications 1 à 4, pour une absorption et un passage par le tissu lymphoréticulaire associé aux muqueuses, dans laquelle les micro-capsules présentent une taille de 1 µm à moins de 5 µm.

6. Utilisation selon l'une des revendications 1 à 4, pour une absorption et une rétention par le tissu lymphoréticulaire associé aux muqueuses, dans laquelle les micro-capsules présentent une taille de 5 µm à moins de 10 µm.

7. Utilisation selon l'une des revendications 1 à 6, destinée à stimuler le système immunitaire d'un être humain ou autre animal.

8. Utilisation selon l'une des revendications 1 à 7, dans laquelle l'agent immunogène est un antigène, un allergène, une lymphokine, une cytokine, une monokine ou un immunorégulateur.

9. Utilisation selon la revendication 8, dans laquelle l'agent immunogène est un antigène de la grippe, un antigène de staphylocoque, un antigène syncytial respiratoire, un antigène de virus para-influenza, un antigène d'Hémophilus influenzae, un antigène de Bordetella pertussis, un antigène de Neisseria gonorrhoeae, un antigène de Streptococcus pneumoniae, un antigène de Plasmodium falciparum, un antigène d'agent pathogène helminthique, un antigène viral, un antigène bactérien, un antigène fongique ou un antigène de protozoose ou un antigène pour une vaccination contre une allergie.

10. Utilisation selon la revendication 9, dans laquelle l'agent immunogène comprend un virus de la grippe ou une entérotoxine staphylococcique B.

11. Utilisation selon l'une des revendications 1 à 10, dans laquelle l'excipient présente une taille et des propriétés chimico-physiques telles que les micro-capsules atteignent effectivement et sont prélevées sélectivement par un tissu lymphoréticulaire associé aux muqueuses.

12. Utilisation selon l'une quelconque des revendications 1 à 11, dans laquelle l'excipient comprend un polymère ou un copolymère.

13. Utilisation selon l'une quelconque des revendications 1 à 12, dans laquelle l'excipient comprend un polymère ou un copolymère biodégradable.

14. Utilisation selon l'une quelconque des revendications 1 à 13, dans laquelle l'excipient est un polymère hydrophobe.

15. Utilisation selon les revendications 10 à 14, dans laquelle l'excipient comprend un poly(DL-lactide-co-glycolide), un poly(L-lactide), un poly(DL-lactide), un poly(glycolide), un copolyoxalate, une polycaprolactone, une poly(lactide-co-caprolactone), un poly(estéramide), un polyorthoester, un acide poly(β-hydroxybutyrique) ou un polyanhydride ou un mélange de ceux-ci.

16. Procédé de réalisation d'une composition destinée à stimuler la réponse immunitaire d'un être humain ou autre animal, comprenant la préparation d'un mélange d'une première quantité de micro-capsules biocompatibles présentant une taille inférieure à approximativement 10 µm et contenant un agent immunogène encapsulé dans un premier excipient biocompatible et d'une seconde quantité de micro-capsules biocompatibles présentant une taille supérieure à 10 µm et contenant un agent immunogène encapsulé dans un second excipient biocompatible, les premières micro-capsules fournissant une réponse immunologique principale et les secondes micro-capsules libérant l'agent contenu dans les secondes micro-capsules de manière pulsée afin de potentialiser une réponse immunologique ultérieure.

17. Procédé selon la revendication 16, dans lequel les premières micro-capsules présentent une taille entre 1 µm et 10 µm.

18. Procédé selon l'une des revendications 16 ou 17, dans lequel le mélange comprend des troisièmes et optionnellement d'autres micro-capsules biocompatibles contenant un agent immunogène, les troisièmes et autres micro-capsules libérant l'agent contenu dans celles-ci de manière pulsée après que les secondes micro-capsules libèrent l'agent contenu dans celles-ci.

19. Procédé selon l'une des revendications 16 à 18, dans lequel deux polymères biodégradables différents sont utilisés en tant que premier et second excipients et dans lequel la vitesse de biodégradation du premier excipient varie par rapport à la vitesse de biodégradation du second excipient.

20. Utilisation d'une composition comprenant un mélange d'une première quantité de micro-capsules biocompatibles présentant une taille inférieure à 10 µm et contenant un agent immunogène encapsulé dans un excipient biocompatible et d'une seconde quantité de micro-capsules biocompatibles présentant une taille supérieure à 10 µm et contenant un agent immunogène encapsulé dans un excipient biocompatible, les premières micro-capsules fournissant une réponse immunologique principale et les secondes micro-capsules libérant l'agent contenu dans les secondes micro-capsules de manière pulsée, afin de préparer une préparation destinée à induire une réponse immunologique dans un être humain ou autre animal.

21. Utilisation selon la revendication 20, dans laquelle les première et seconde et optionnellement autres micro-capsules sont administrées dans une préparation.

22. Utilisation destinée à la préparation d'une composition combinée comprenant des premières micro-capsules biocompatibles comprenant un agent immunogène encapsulé dans un excipient biocompatible et présentant une taille entre 1 µm et 10 µm en vue d'une administration à un être humain ou autre animal par une première voie et des secondes micro-capsules biocompatibles comprenant l'agent immunogène encapsulé dans un excipient biocompatible et présentant une taille entre 1 µm et 10 µm en vue d'une administration à un être humain ou autre animal par une seconde voie différente de la première voie afin de potentialiser la réponse immunitaire d'un être humain ou autre animal.
